(19) Europäisches Patentamt
European Patent Office
Office européen des brevets

(11) EP 3 473 098 A1

(12) EUROPEAN PATENT APPLICATION
published in accordance with Art. 153(4) EPC

(43) Date of publication:
24.04.2019 Bulletin 2019/17

(21) Application number: 17813677.6

(22) Date of filing: 19.06.2017

(51) Int Cl.:
*A01N 37/42* (2006.01) *A01N 55/02* (2006.01)
*A01P 21/00* (2006.01)

(86) International application number:
PCT/RU2017/000432

(87) International publication number:
WO 2017/217892 (21.12.2017 Gazette 2017/51)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 17.06.2016 RU 2016123990

(71) Applicants:
- **Gorbunov, Sergei Valeryevich**
  **Moscow 127055 (RU)**
- **Starikov, Ivan Aleksandrovich**
  **g. Murmansk 183025 (RU)**

(72) Inventor: **GORBUNOV, Sergei Valeryevich**
**g. Murmansk 183025 (RU)**

(74) Representative: **ABG Intellectual Property, S.L.**
**Avenida de Burgos, 16D**
**Edificio Euromor**
**28036 Madrid (ES)**

# (54) COMPOSITION FOR REGULATING PLANT GROWTH, METHOD FOR TREATING PLANTS THEREWITH, AND ACTIVE INGREDIENT THEREOF

(57) A composition for regulating plant growth, the active ingredient of which is an ester of oxaloacetic acid or a salt thereof, or derivatives thereof, wherein the ester of oxaloacetic acid has the given formula, where $R^1$ and $R^2$ are independently selected from a series of $C_1$-$C_{18}$ alkyl groups, and wherein the content of the active ingredient corresponds to a concentration of from $10^{-11}$ M to $10^{-3}$ M. The active ingredients and compositions for regulating plant growth allow for treating plants in order to stimulate the growth thereof and achieve such advantages as increased yields, improved plant quality, improved nutrient absorption and enhanced stress resistance, while allowing such advantages to be achieved using a relatively small amount of an active ingredient(s) which is/are biodegradable and environmentally friendly.

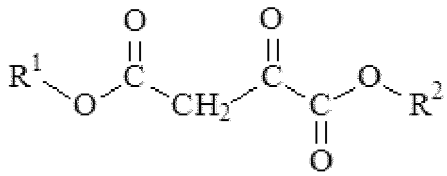

EP 3 473 098 A1

## Description

PERTINENT ART

The present disclosure relates to plant growth regulators and their active ingredients, and more specifically to plant growth regulator compounds and compositions, and methods of treating plants with such compounds and compositions. The compounds, compositions, and methods of the present disclosure can be used in agriculture, in private household farming, in plant growing and crop farming, to mediate favorable results in a wide variety of plants, such as to stimulate plant growth, increase plant yields, and improve the quality of plant products.

BACKGROUND ART

There are many different plant growth regulators, but most of them are efficient only for a small group of plants or for a certain stage of growth. Moreover, these regulators, as a rule, are either expensive or low-active, or require high dosages. Known plant growth regulators are of widely varying types, and may be employed to stimulate various individual plant growth processes, including seed germination, root formation, stem elongation, taproot elongation, etc. Among currently utilized plant growth regulator formulations, a broad spectrum of active ingredients is utilized, including auxins, gibberellins, cytokinins, brassinosteroids, silatranes, melissyl alcohol, arachidonic acid, 2,6-dimethylpyridine N-oxide, melamine bis(hydroxymethyl)phosphinate, amber acid, and others.

There are plant growth regulators in the form of an aqueous chelate complex of transition metal with two anions of dimethyl ester of oxaloacetic acid, as used at a concentration of 0.0001-0.01% (approx. $2.5.10^{-6}$-$2.5.10^{-4}$ M). They may for example contain as an active ingredient: *trans*-diaqua-*trans*-bis[1-oxy-1,2-di(methoxycarbonyl)ethenato]zinc, as described in Invention Patent UA19841 (IPC A01N 55/02, 1/00, 13/00 C07F A01P), issued December 25, 1997; *trans*-diaqua-*trans*-bis[1-oxy-1,2-di(methoxycarbonyl)ethenato]manganese(II), as described in Invention Patent UA19812 (IPC A01N 55/02, 1/00, 13/00 C07F A01P), issued December 25, 1997; or *trans*-diaqua-*trans*-bis[1-oxy-1,2-di(methoxycarbonyl)ethenato]nickel(II), as described in Invention Patent UA19840 (IPC A01N 55/02, 21/00, 15/00 C07F A01P), issued December 25, 1997.

A common disadvantage of these plant growth regulators and their active ingredients resides in there use requiring relatively high effective concentrations of the active ingredient(s) that significantly exceed the content of the most active natural hormones in the plants (for example, brassinosteroid concentrations may range up to $10^{-11}$ M). Also these plant growth regulators and their active ingredients have quite a narrow range of growth stimulating effects, such as being limited only to promotion of elongation of the main stem and taproot of the germinants. There are no known publicly available data on the effect of these prior art solutions on yield, and such effect is not obvious from the prior art.

There is known a plant growth regulator in the form of an aqueous solution of an active ingredient, used at concentrations of $10^{-8}$ to $10^{-4}$ M (0.0016 to 16 mg/l), where the dimethyl ester of 2-aminofumaric acid is used as an active ingredient, as described in Invention Patent RU2184450 (IPC A01N37/44), issued July 10, 2002.

A significant disadvantage of this prior art solution is that the synthesis of the dimethyl ester of 2-aminofumaric acid requires dimethyl acetylenedicarboxylate, which has a strong tear and blistering effect, which in turn significantly complicates the process of its production and increases its hazardous character.

A formulation for increasing a growth characteristic of a plant, increasing nutrient use efficiency of a plant, or improving a plant's ability to overcome stress, in the form of an aqueous solution of an active ingredient at concentrations of $10^{-7}$ to $10^{-2}$ M, comprising ketosuccinamate, a derivative thereof, or a salt thereof as an active ingredient, is described in U.S. Patent Application Publication 20150051072 (IPC A01N43/34, A01N37/18, A01N43/40, A01N43/36, A01N43/62, C05G3/0000, issued February 19, 2015. Ketosuccinamate is selected as a prototype of the invention of this disclosure.

The disadvantage of formulations of the type described in U.S. Patent Application Publication 20150051072 is a limited range of growth stimulating effects, such as increased germination of seeds, foliar biomass growth enhancement, increase in tuber mass (of soy), and increase of bushiness and number of grains in a head (of wheat). There is also no specific data given in such reference on the effect of such formulations on yield increase. In addition, the use of these formulations specifically for stimulation of growth processes involves relatively high preferred concentrations of ketosuccinamate (or its salts) in aqueous solutions ($10^{-4}$ to $10^{-2}$ M), which entails significant consumption of the active ingredient in the use of the corresponding formulation.

SUMMARY

The present disclosure relates to plant growth regulator compounds and compositions, and methods for treating plants with same, to achieve beneficial effects such as the stimulation of plant growth, increases in plant yields, and improvements in the quality of plant products.

In one aspect, the disclosure relates to a plant growth regulator composition, comprising, as active ingredient,

at least one of an oxaloacetic acid ester, and salts and derivatives thereof, wherein the oxaloacetic acid ester has the formula:

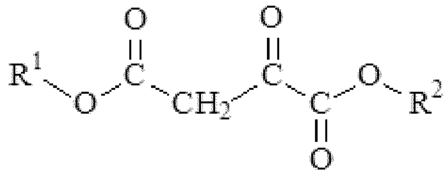

,

wherein $R^1$ and $R^2$ are each independently selected from $C_1$-$C_{18}$-alkyl groups, and wherein the active ingredient content corresponds to a concentration from $10^{-11}$ M to $10^{-3}$ M.

In another aspect, the disclosure relates to a method for treating plants to increase their growth, comprising applying to said plants or to a locus containing said plants a plant growth regulator composition of the present disclosure.

A further aspect of the invention relates to an active ingredient of a plant growth regulator, said active ingredient being an ester of oxaloacetic acid of the general formula:

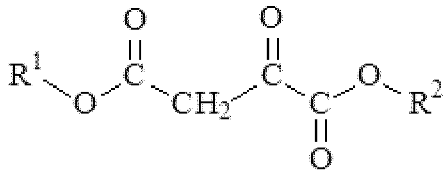

,

where $R^1$ and $R^2$ are each independently selected from $C_1$-$C_{18}$-alkyl groups, and salts or other derivatives of this ester can also be used.

Other aspects, features, and advantages of the disclosure will be more fully apparent from the ensuing disclosure and appended claims.

DESCRIPTION

As used herein and in the appended claims, the singular forms "a", "and", and "the" include plural referents unless the context clearly dictates otherwise.

The disclosure, as variously set out herein in respect of features, aspects and embodiments thereof, may in particular implementations be constituted as comprising, consisting, or consisting essentially of, some or all of such features, aspects and embodiments, as well as elements and components thereof being aggregated to constitute various further implementations of the disclosure. The disclosure correspondingly contemplates such features, aspects and embodiments, or a selected one or ones thereof, in various permutations and combinations, as being within the scope of the present disclosure.

As used herein, the identification of a carbon number range, e.g., in $C_1$-$C_{12}$ alkyl, is intended to include each of the component carbon number moieties within such range, so that each intervening carbon number and any other stated or intervening carbon number value in that stated range, is encompassed, it being further understood that sub-ranges of carbon number within specified carbon number ranges may independently be included in smaller carbon number ranges, within the scope of the disclosure, and that ranges of carbon numbers specifically excluding a carbon number or numbers are included in the disclosure, and sub-ranges excluding either or both of carbon number limits of specified ranges are also included in the disclosure. Accordingly, $C_1$-$C_{18}$ alkyl is intended to include methyl, ethyl, propyl, butyl, pentyl, hexyl, heptyl, octyl, nonyl, decyl, undecyl, dodecyl, etc., including straight chain as well as branched groups of such types. It therefore is to be appreciated that identification of a carbon number range, e.g., $C_1$-$C_{18}$, as broadly applicable to a substituent moiety, enables, in specific embodiments of the disclosure, the carbon number range to be further restricted, as a sub-group of moieties having a carbon number range within the broader specification of the substituent moiety. By way of example, the carbon number range e.g., $C_1$-$C_{18}$ alkyl, may be more restrictively specified, in particular embodiments of the invention, to encompass sub-ranges such as $C_1$-$C_4$ alkyl, $C_2$-$C_8$ alkyl, $C_2$-$C_4$ alkyl, $C_3$-$C_5$ alkyl, $C_4$-$C_{14}$ alkyl or any other sub-range within the broad carbon number range. In other words, a carbon number range is deemed to affirmatively set forth each of the carbon number species in the range, as to the substituent, moiety,

or compound to which such range applies, as a selection group from which specific ones of the members of the selection group may be selected, either as a sequential carbon number sub-range, or as specific carbon number species within such selection group.

The same construction and selection flexibility is applicable to stoichiometric coefficients and numerical values specifying the number of atoms, functional groups, ions or moieties, as to specified ranges, numerical value constraints (e.g., inequalities, greater than, less than constraints), as well as oxidation states and other variables determinative of the specific form, charge state, and composition applicable to chemical entities within the broad scope of the present disclosure.

Any of the various selection groups herein specified may be delimited for purposes of specifying the selection species thereof, by specific exclusion of one or more species of such group.

The plant growth regulator compounds and compositions of the present disclosure enable treatment of plants to increase their growth as well as to achieve benefits such as increases in yields, and improvements in plant quality, nutrient utilization, and stress tolerance, while permitting such benefits to be realized with relatively small applied amounts of the plant growth regulator active ingredient(s) which are readily biodegradable and environmentally benign.

The plant growth regulator compounds utilized as active ingredients in plant growth regulator compositions of the present disclosure include oxaloacetic acid esters, and salts thereof, and derivatives thereof having phytologically beneficial plant growth regulator characteristics.

The present disclosure therefore relates in a primary aspect to a plant growth regulator composition, comprising, as active ingredient, at least one of an oxaloacetic acid ester, and salts and derivatives thereof, wherein the oxaloacetic acid ester has the formula:

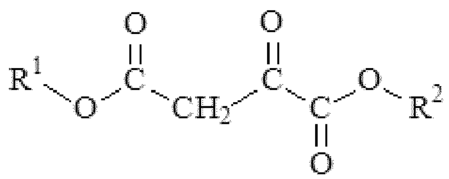

,

wherein $R^1$ and $R^2$ are each independently selected from $C_1$-$C_{18}$-alkyl groups, and wherein the active ingredient content corresponds to a concentration from $10^{-11}$ M to $10^{-3}$ M.

In various embodiments, the active ingredient comprises a sodium salt of dimethyl oxaloacetate:

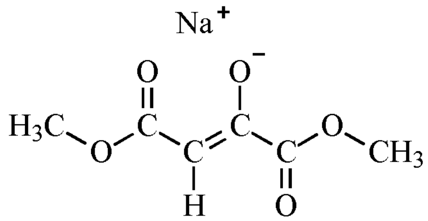

In other embodiments, the active ingredient comprises a sodium salt of diethyl oxaloacetate:

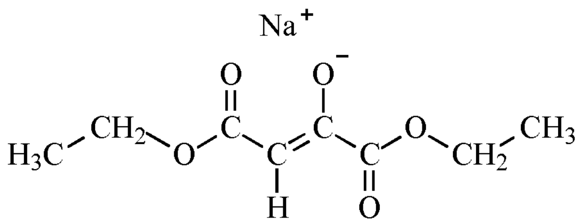

The plant growth regulator composition may comprise a carrier or delivery medium for the active ingredient, e.g., an organic solvent, such as dimethyl sulfoxide, or water or other aqueous medium.

The disclosure relates in another aspect to a method for treating plants to increase their growth, comprising applying to said plants or to a locus containing said plants the plant growth regulator composition of the present disclosure. The plants may for example comprise any of grains, legumes, fiber producing plants, oil producing plants, tuber producing

plants, starch producing plants, grasses, vines, fruits, vegetables, flowering plants, and trees. In other embodiments, the plants may comprise any of sunflower, soybean, alfalfa, cucumber, tomato, wheat, corn, cotton, rice, pumpkin, sugarcane, potato, barley, coffee, bean, kidney bean, soybean, french bean, runner bean, haricot, peas, chickpea, lentil, millet, onion, clover, melilot, batata, yam, Jerusalem artichoke (Helianthus tuberosus), cassava, artich oke, oat, rice, peanut, maize, sorghum, radish, horse, turnip, carrot, canola, rapeseed, flax, sesame, asparagus, lettuce, legume, grape, berry, vine, orange, nut, coconut tree, tobacco, pepper, red pepper, sweet pepper, mustard, buckwheat, eggplant, vegetable marrow, zucchini, melon, watermelon, pineapple, banana, papaya, avocado, kiwi, panicgrass, beet, dill, fennel, mint, cabbage, nappacabbage, cilantro, ginger, parsley, spinach, ruccola, celery, Brussel sprouts, mango, strawberry, cauliflower, oilpalms (Elaeis), hops, cannabis, cocoabean, salvia, aster, China aster (Callistephus chinensis), dragon flowers (Antirrhinum), carnation (Dianthus caryophyllus), rose, dahlia, chrysanthemum, tulpin, narcissus, delphinium, iris, clematis, peony, phlox, rhododendron, hyacinthus, cyclamen, petunia, lilium, and orchids.

The present disclosure relates in another aspect to an active ingredient of a plant growth regulator, said active ingredient being an ester of oxaloacetic acid of the general formula:

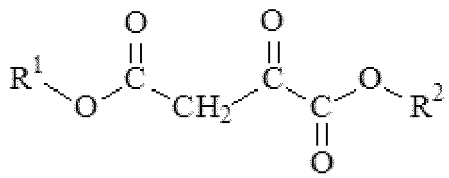

,

where $R^1$ and $R^2$ are each independently selected from $C_1$-$C_{18}$-alkyl groups, and salts or other derivatives of this ester, and mixture of thereof can also be used.

It will be appreciated from the foregoing that the plant growth regulator compounds and compositions of the present disclosure comprise a variety of compound species and compositions, which may be formulated in any suitable manner to provide growth-promoting treatments to any of a wide variety of plants and plant species.

Salts of the acids of the oxaloacetic acid esters of the disclosure may be of any suitable type, e.g., sodium salts, calcium salts, iron salts, copper salts, magnesium salts, potassium salts, ammonium salts, etc.

The term "derivatives" as used herein includes any suitable plant growth regulator compounds (the term "compounds" herein being construed as inclusive of salts and complexes) which retain an oxaloacetic acid carbon skeleton, to which various substituent(s) and/or chemical moiety/moieties may be covalently or coordinately bound. Such derivatives can be described by the following general formula:

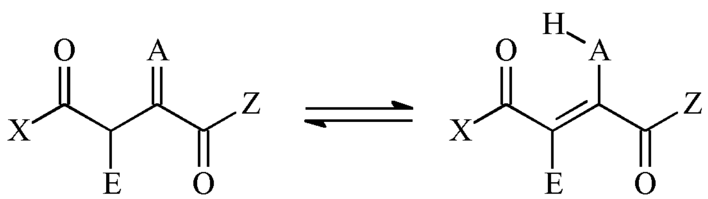

wherein A, E, X and Z are each independently take certain values, namely:

A = O, S, NH, N-OH, N-$NH_2$;

X = OH, $NH_2$, O-R (where R = $C_1$-$C_{18}$-alkyl, $C_1$-$C_{18}$-alkenyl, $C_1$-$C_{18}$-alkynyl, $C_3$-$C_6$-cycloalkyl, wherein one or more hydrogen atoms inside the cycloalkyl may be substituted by $C_1$-$C_4$-alkyl groups, benzyl), NH-R' (wherein R' = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1'}R^{2'}$ (wherein $R^{1'}$ and $R^{2'}$ are independently take values: $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholyl, $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl (including ring substituted by one or more chloro), 2-phenethyl (including ring substituted by one or more chlorine), 2-phenylvinyl (including ring substituted by one or more chloro), naphthyl, indolyl, furanyl, thiophenyl;

Z = OH, $NH_2$, O-R" (where R" = $C_1$-$C_{18}$-alkyl, $C_1$-$C_{18}$-alkenyl, $C_1$-$C_{18}$-alkynyl, $C_3$-$C_6$-cycloalkyl, wherein one or more

hydrogen atoms within the cycloalkyl may be substituted by $C_1$-$C_4$-alkyl groups, benzyl), NH-R* (wherein R* = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1}"R^{2}"$ (wherein $R^{1}"$ and $R^{2}"$ each independently take values: $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholinyl;

E = H, CN, halogen (F, Cl, Br, I), O-R'" (wherein R'" = $C_1$-$C_4$-alkyl, allyl, propargyl, $C_3$-$C_6$-cycloalkyl, benzyl), S-R'" (wherein R'" = $C_1$-$C_4$-alkyl, allyl, propargyl, $C_3$-$C_6$-cycloalkyl, benzyl), O-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), S-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl, naphthyl, indolyl, furanyl, tetrahydrofuranyl, thiophenyl, tetrahydrothiophenyl;

also X and E may be connected forming a 5-7-membered cycle through the following bridges (from X-terminus to E-terminus): $-(CH_2)_m-$ (wherein m = 3-5), - $(CH_2)_n$-(C=O)- (where n = 2-4), $-O-(CH_2)_n-$ (where n = 2-4), $-NH-(CH_2)_n-$ (where n = 2-4), -CH*-$(CH_2)_k$-CH*- (wherein k = 1-2, and CH* carbons are connected to each other via a $-(CH_2)_j-$ bridge, where j = 1-2, regardless k), as any C-C bond within the said bridges may be fuse with benzene ring (wherein the benzene ring may be substituted by one or more identical or different functional groups of the following: methyl, ethyl, propyl, butyl, hydroxy, methoxy, ethoxy, halogen, nitro, trifluoromethyl), also one or more hydrogen atoms within said bridges (including NH, if present) may be further substituted by $C_1$-$C_4$-alkyl group;

and E and Z may be joined to form a 5-6-membered ring through following bridges (from Z-terminus to E-terminus): $-NH-(CH_2)_r-$ (wherein r = 1-2), and one or more of the hydrogen atoms inside said bridges (including NH) may be further substituted by $C_1$-$C_4$-alkyl group;

herewith oxaloacetic acid derivatives exclude compounds where A, E, X, Z simultaneously have the following values: A = O, E = H, X = $NH_2$ and Z = OH in the first case; A = NH, E = H, X = Z = $OCH_3$ in the second case; A = NH, E = H, X = $OC_2H_5$ and Z = $OCH_3$ in the third case; A = NH, E = H, X = $OCH_3$, Z = OH/OK in the fourth case; A = NH, E = H, X = $OCH_3$, Z = $NH_2$ in the fifth case; A = NH, E = H, X = $OC_2H_5$, Z = OH/OK in the sixth case; A = NH, E = H, X = $OC_2H_5$, Z = $NH_2$ in the seventh case; A = NH, E = H, X = $OCH_3$, Z = $-NH-CH_2-Ph$ (wherein Ph = phenyl) in the eighth case;

herewith derivatives of oxaloacetic acid also include salts and complexes with metals for all permissible compounds mentioned above as suitable derivatives, except chelate complexes consisting of two anions of dimethyl ester of oxaloacetic acid, and zinc / manganese / nickel.

Illustrative derivatives include the following compounds 1-70, in which various of the compounds are shown with respect to tautomeric forms thereof, e.g., keto-enol tautomeric forms:

1. 4-Ethoxy-2,4-dioxobutanoic acid:

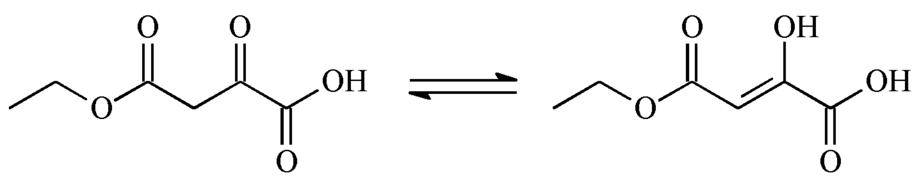

2. 4-Ethoxy-3,4-dioxobutanoic acid:

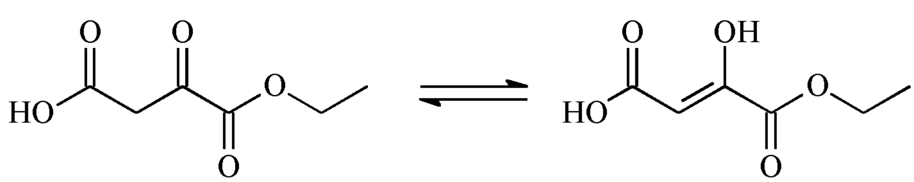

3. Dicyclopentyl oxalacetate:

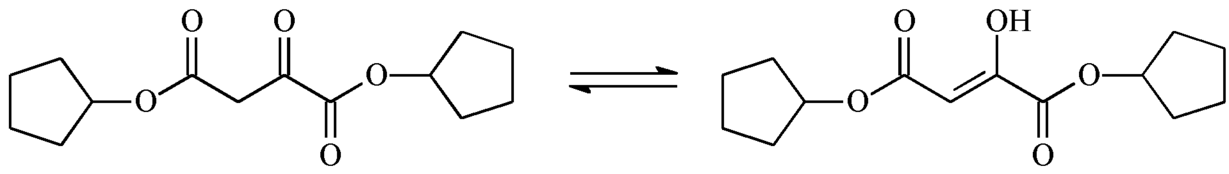

4. Ethyl 4-(dimethylamino)-2,4-dioxobutanoat:

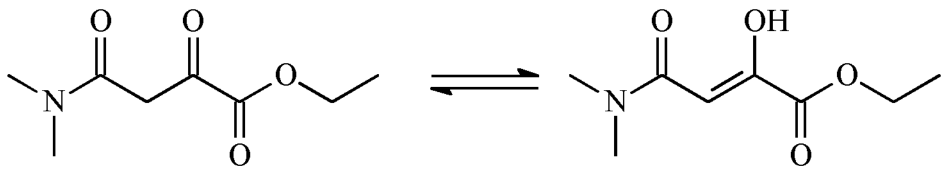

5. $N^1$,$N^1$,$N^4$,$N^4$-tetramethyl-2-oxobutanediamide:

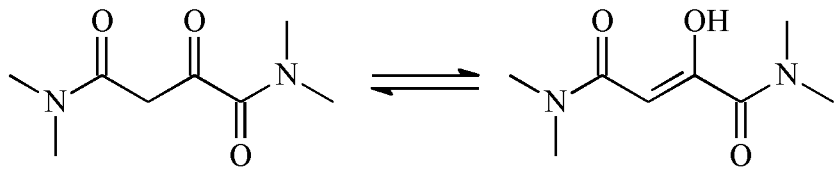

6. $N^4$,$N^4$-diethyl-2-oxobutanediamide:

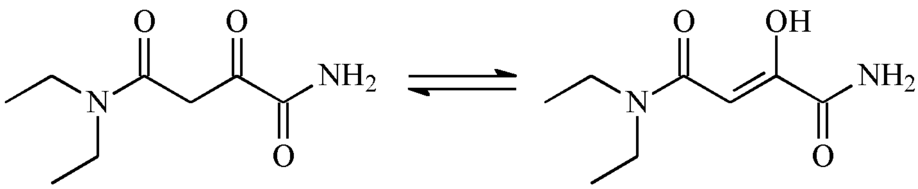

7. $N^1$,$N^4$-dimethyl-2-oxobutanediamide:

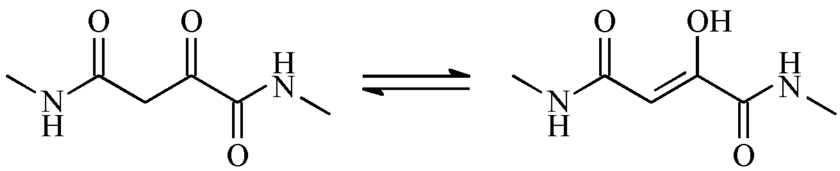

8. $N^1N^4$-dibenzyl-2-oxobutanediamide:

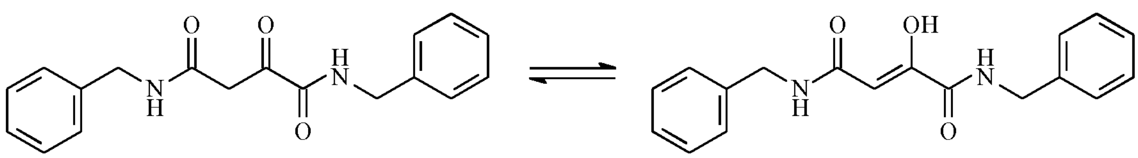

9. Methyl 4-(4-methylanilino)-3,4-dioxobutanoate:

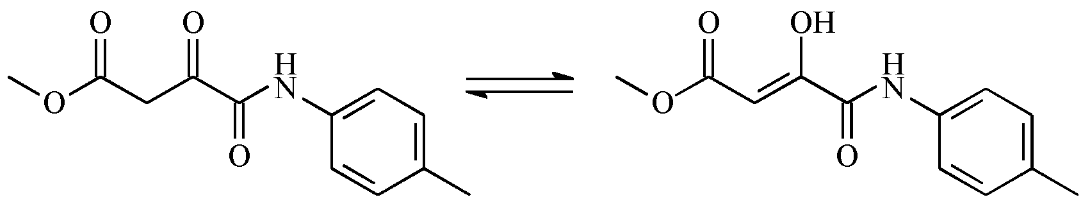

10. Ethyl 2,4-dioxo-4-(pyrrolidin-1-yl)butanoate:

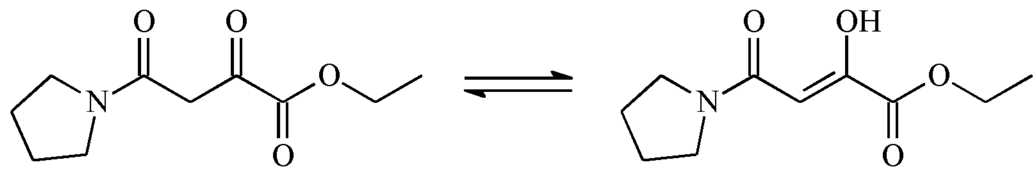

11. Ethyl 4-(morpholin-4-yl)-2,4-dioxobutanoate:

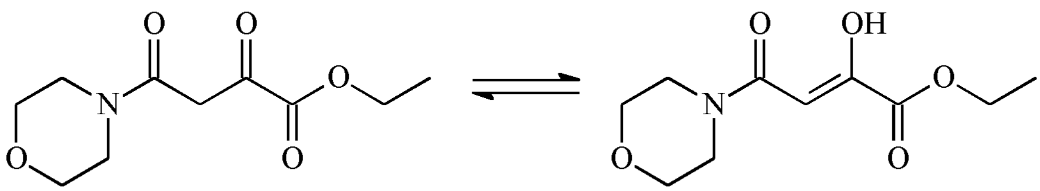

12. Ethyl 4-(4-hydroxypiperidin-1-yl)-2,4-dioxobutanoate:

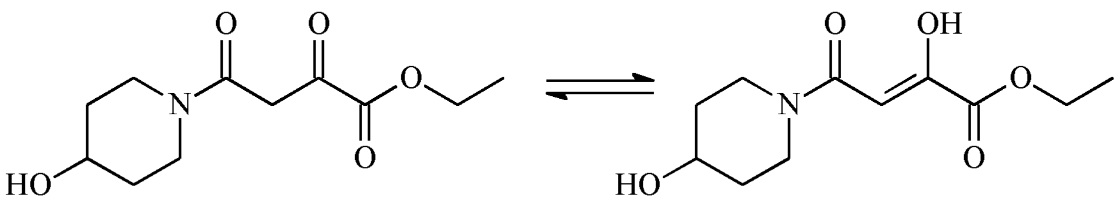

13. $N^4$-(2-hydroxyethyl)-$N^1$,$N^1$,$N^4$-trimethyl-2-oxobutanediamide:

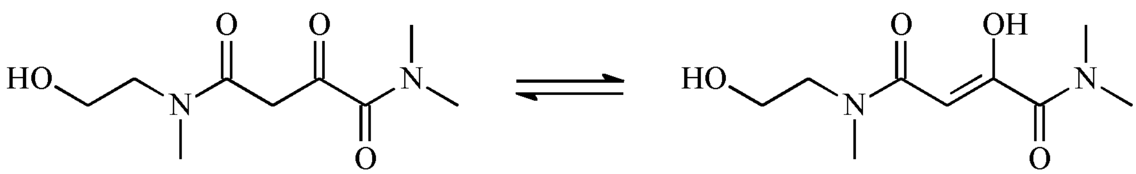

14. $N^4$-(2-methoxyethyl)-$N^1$,$N^1$,-dimethyl-2-oxobutanediamide:

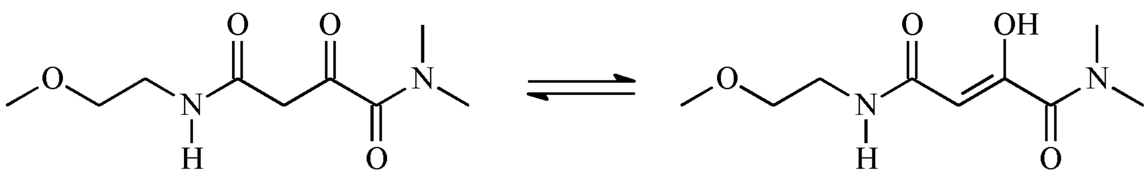

15. Ethyl (1-methyl-2-oxopyrrolidin-3-yl)(oxo)acetate:

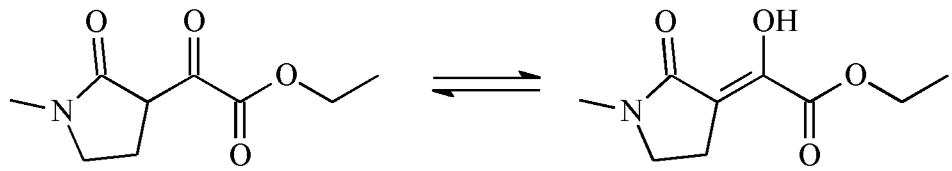

16. Ethyl (1-methyl-2-oxopiperidin-3-yl)(oxo)acetate:

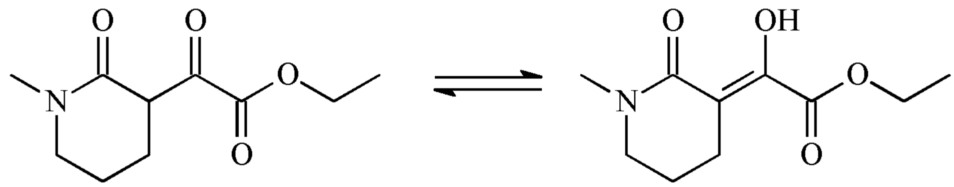

17. Methyl oxo(2-oxooxan-3-yl)acetate:

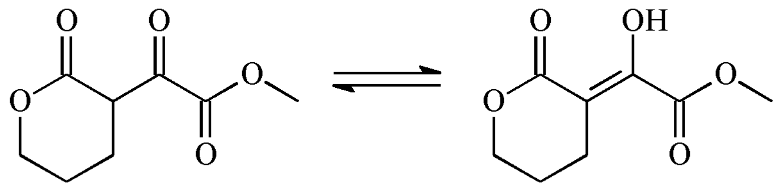

18. Methyl 4,5-dioxopyrrolidine-3-carboxylate:

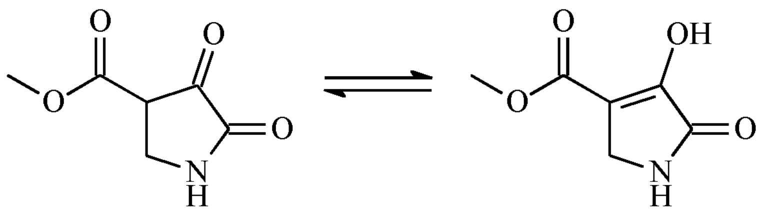

19. Ethyl 2,4-dioxovalerate:

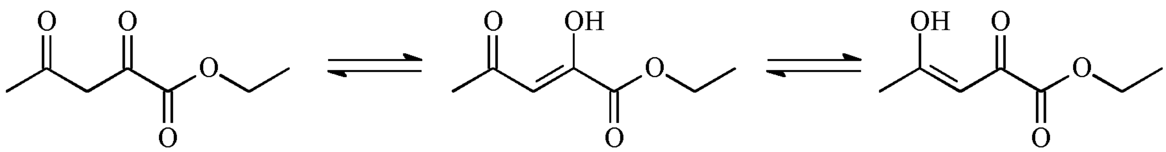

20. Ethyl 8-methyl-2,4-dioxononanoate:

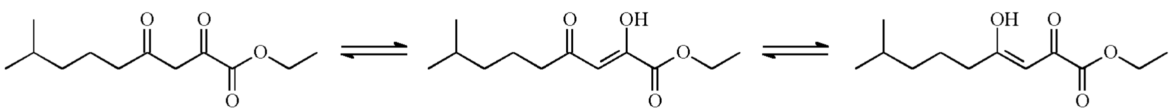

21. Ethyl 8-methyl-2,4-dioxonon-7-enoate:

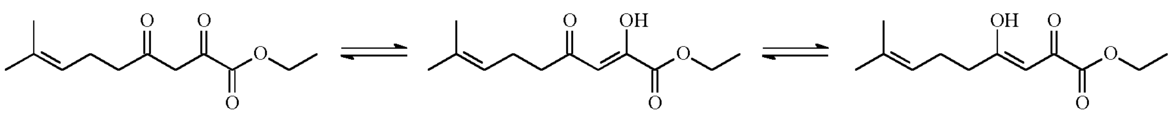

22. Ethyl 4-cyclopropyl-2,4-dioxobutanoate:

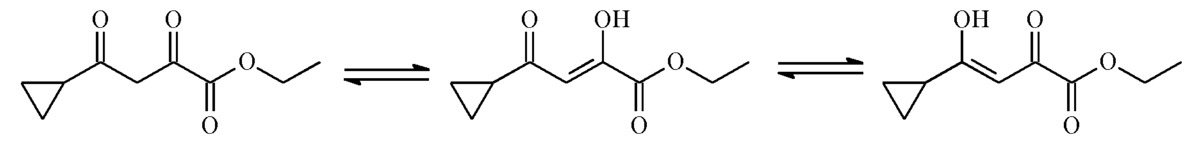

23. Ethyl 4-cyclopentyl-2,4-dioxobutanoate:

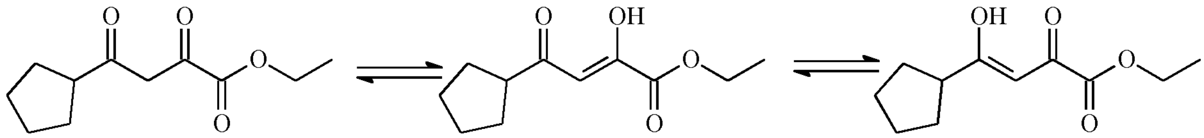

24. Ethyl 2,4-dioxo-4-(2,2,6-trimethylcyclohexyl)butanoate:

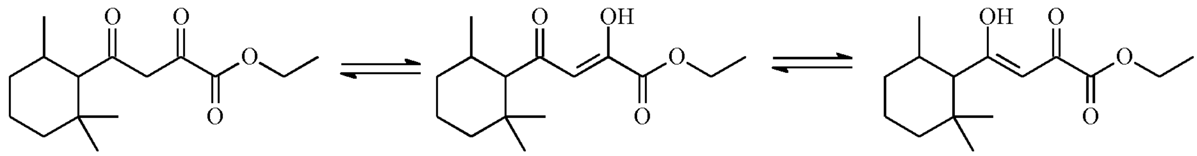

25. Ethyl (5*E*)-2,4-dioxo-6-phenylhex-5-enoate:

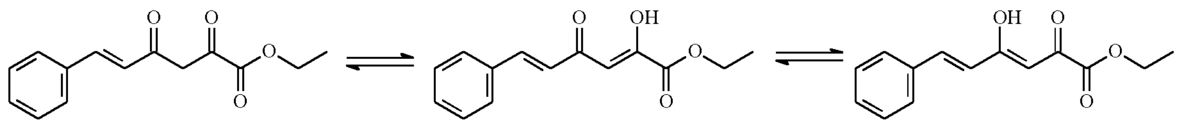

26. Ethyl 2,4-dioxo-4-phenylbutanoate:

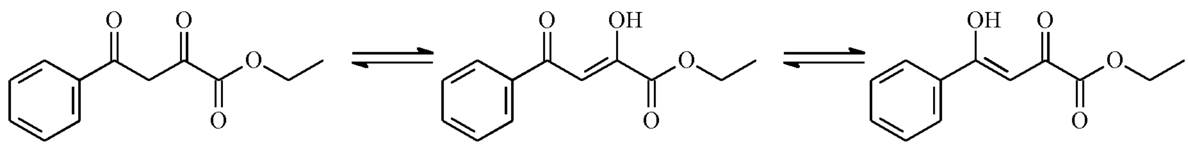

27. Ethyl 4-(4-methylphenyl)-2,4-dioxobutanoate:

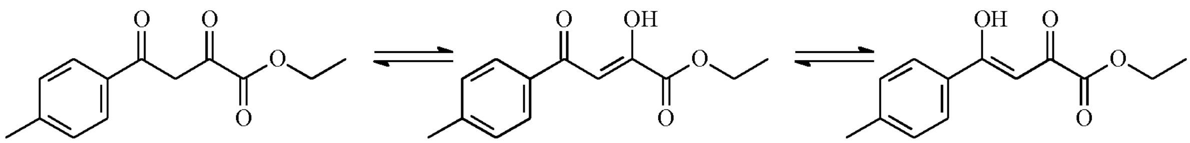

28. Ethyl 4-(4-nitrophenyl)-2,4-dioxobutanoate:

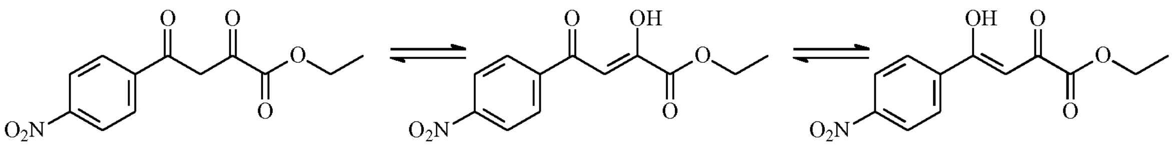

29. 2,4-Dioxo-4-[3-(trifluoromethyl)phenyl]butanoic acid:

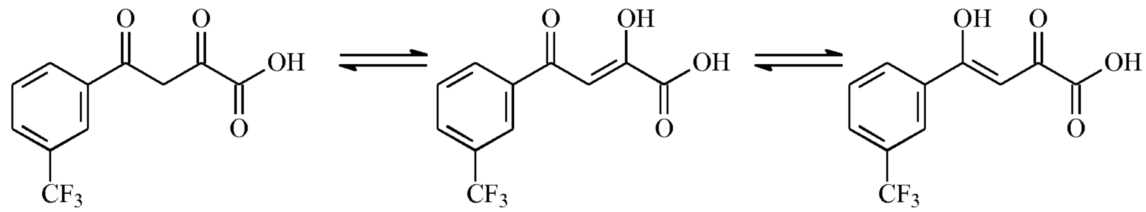

30. Ethyl 4-(2,4-dichlorophenyl)-2,4-dioxobutanoate:

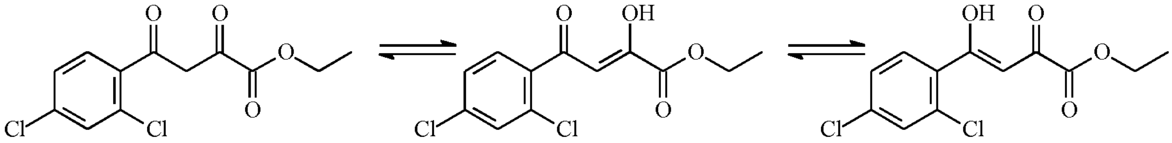

31. Ethyl 4-(3-fluoro-4-methoxyphenyl)-2,4-dioxobutanoate:

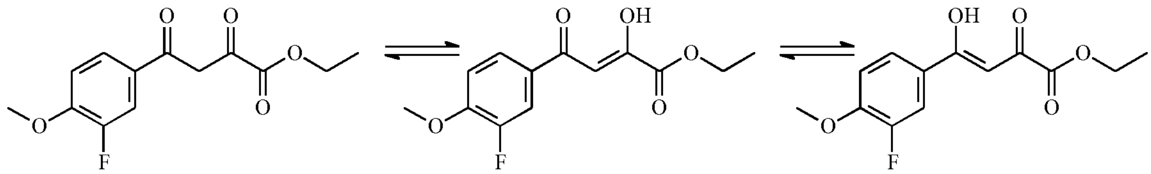

32. Methyl 4-(furan-2-yl)-2,4-dioxobutanoate:

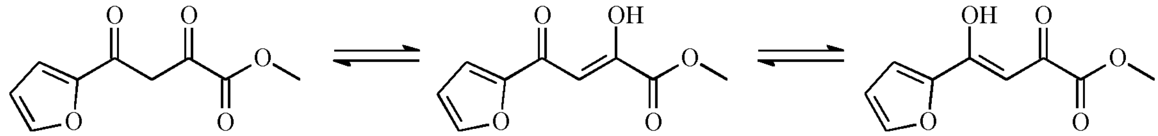

33. Ethyl 2,4-dioxo-4-(thiophen-2-yl)butanoate:

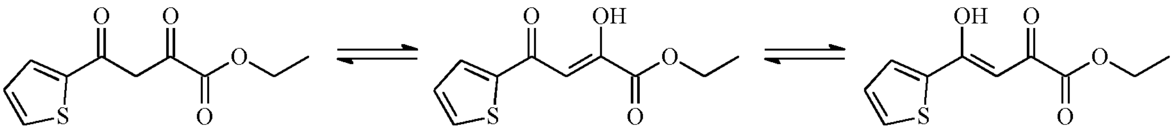

34. Ethyl 4-(naphtalen-1-yl)-2,4-dioxobutanoate:

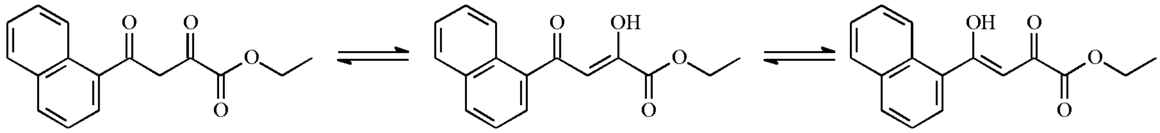

35. Ethyl 4-(1*H*-indol-3-yl)-2,4-dioxobutanoate:

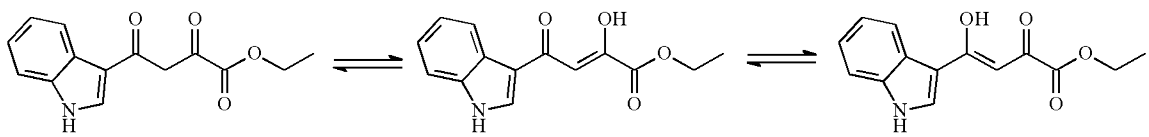

36. Dimethyl 2-cyano-3-oxobutanedioate:

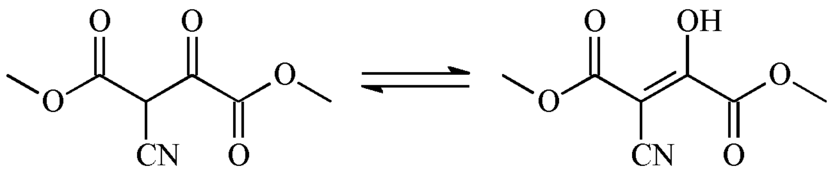

37. Dimethyl 2-chloro-3-oxobutanedioate:

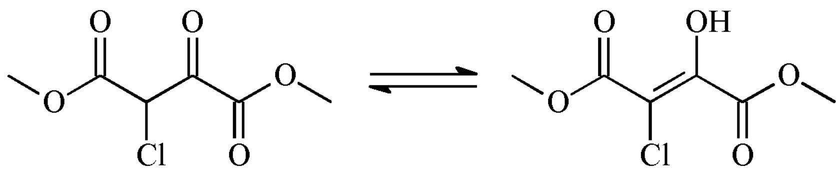

38. Diethyl 2-fluoro-3-oxobutanedioate:

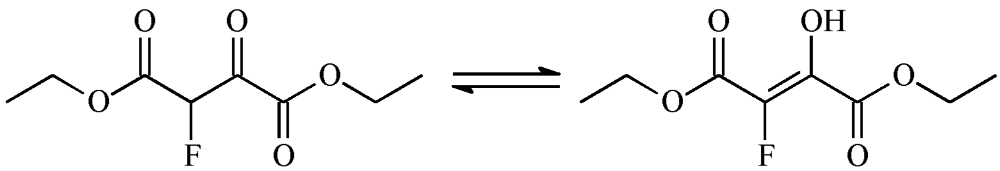

39. Diethyl 2-methoxy-3-oxobutanedioate:

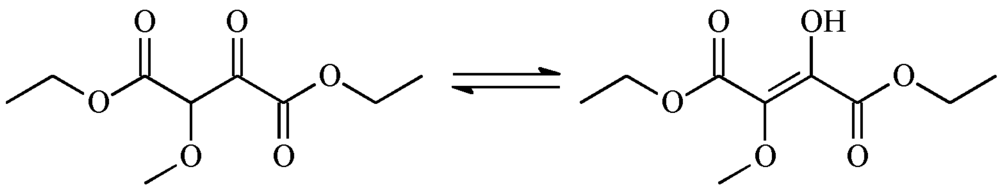

40. Diethyl 2-(butylsulfanyl)-3-oxobutanedioate:

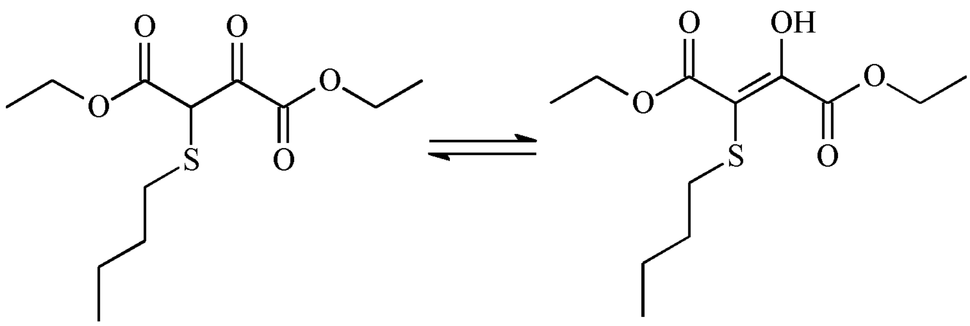

41. Dimethyl 2-methyl-3-oxobutanedioate:

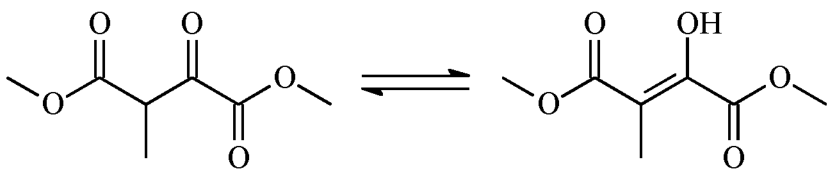

42. Diallyl 2-ethyl-3-oxobutanedioate:

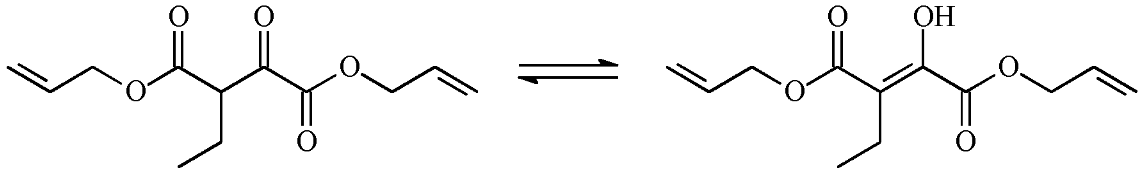

43. Diethyl 2-(2-methylprop-1-en-1-yl)-3-oxobutanedioate:

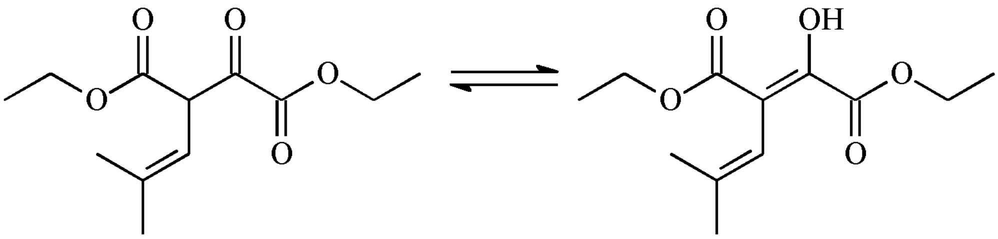

44. Dimethyl 2-cyclohexyl-3-oxobutanedioate:

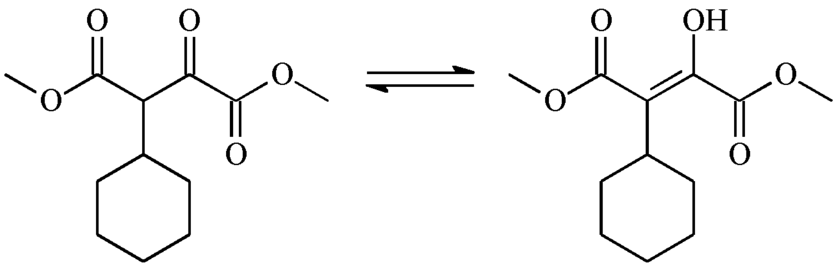

45. Diethyl 2-oxo-3-(oxolan-2-yl)butanedioate:

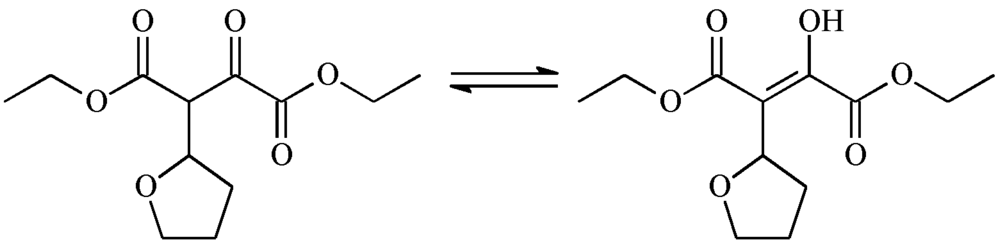

46. Diethyl 2-oxo-3-phenylbutanedioate:

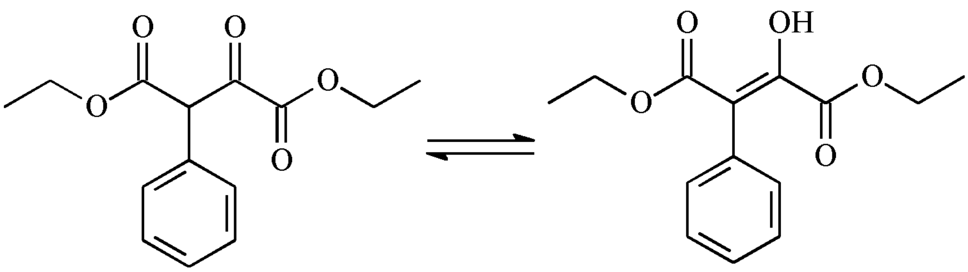

47. Diethyl 2-(4-methoxyphenyl)-3-oxobutanedioate:

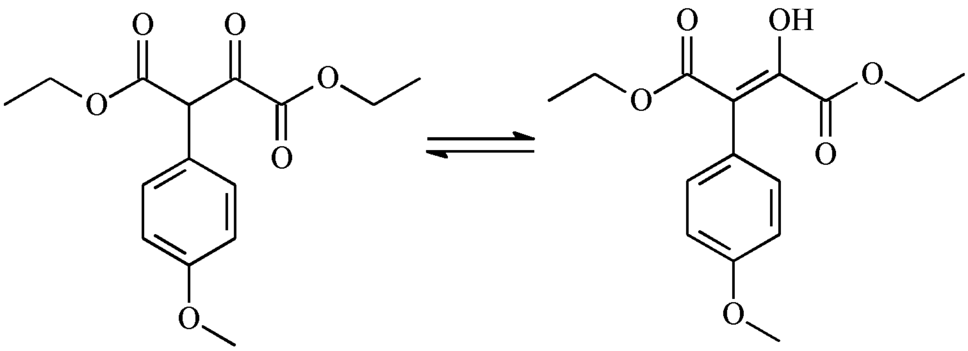

48. Diethyl 2-(naphthalen-1-yl)-3-oxobutanedioate:

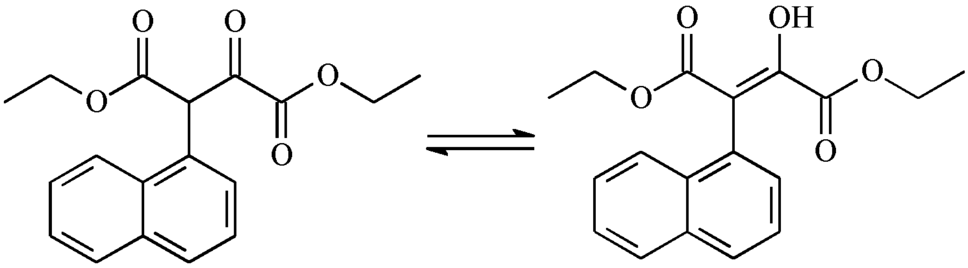

49. Dimethyl 2-(1*H*-indol-3-yl)-3-oxobutanedioate:

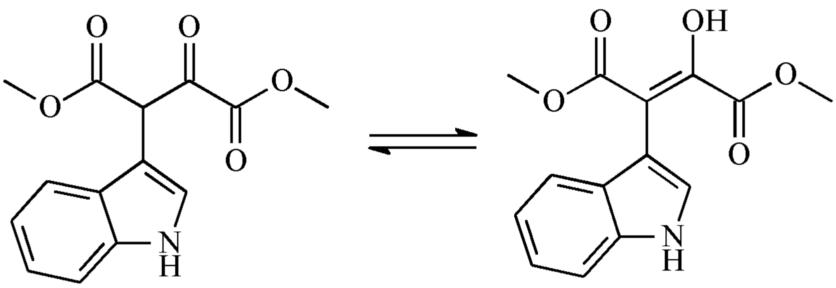

50. Diethyl 2-(4-chlorophenoxy)-3-oxobutanedioate:

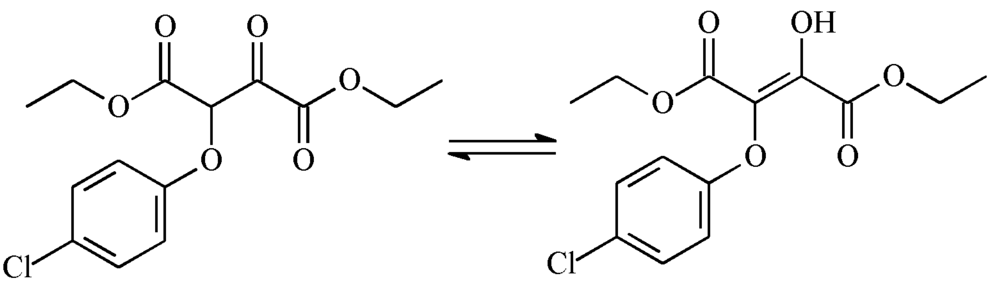

51. Diethyl 2-[(4-methylphenyl)sulfanyl]-3-oxobutanedioate:

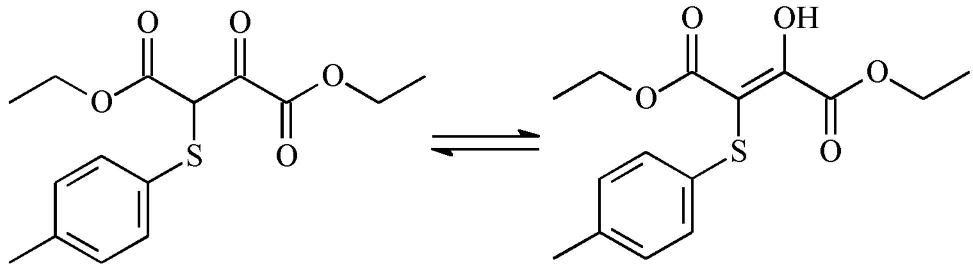

52. Ethyl oxo(2-oxocyclohexyl)acetate:

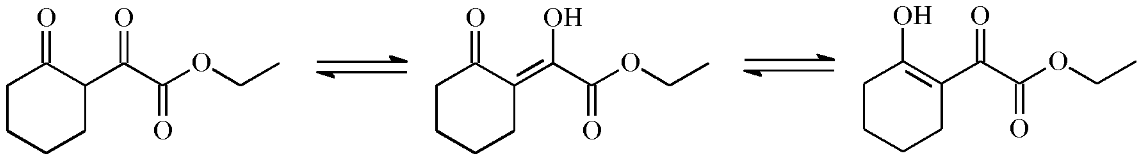

53. Ethyl (2,6-dioxocyclohexyl)(oxo)acetate:

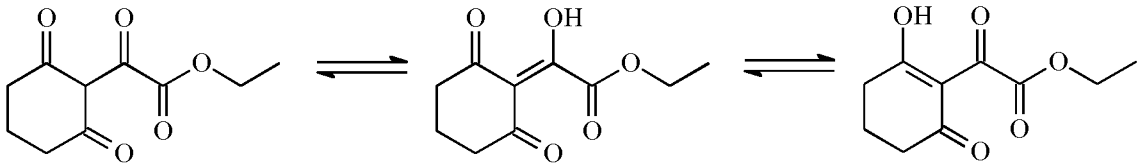

54. Ethyl oxo(1-oxo-1,2,3,4-tetrahydronaphthalen-2-yl)acetate:

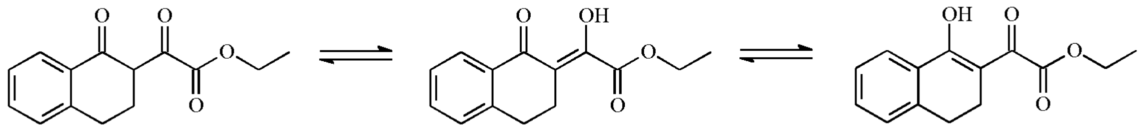

55. Ethyl oxo(3-oxobicyclo[2.2.1]heptan-2-yl)acetate:

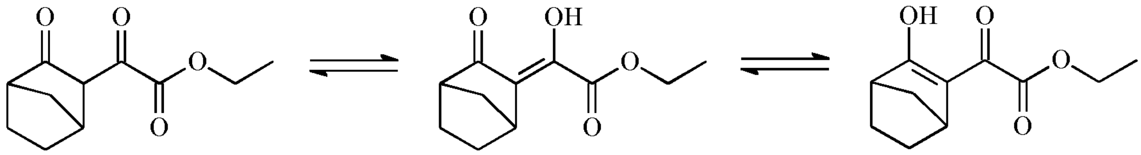

56. Ethyl oxo(2-oxocyclopentyl)acetate:

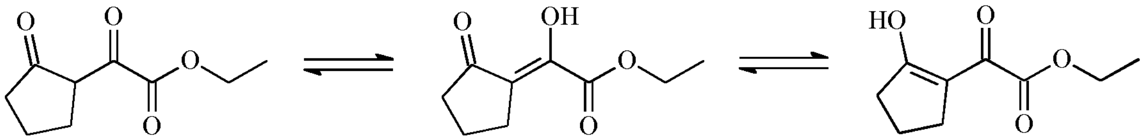

57. Ethyl oxo(1-oxo-2,3-dihydro-1*H*-inden-2-yl)acetate:

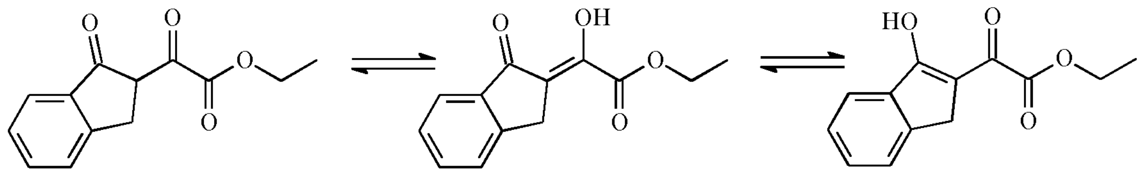

58. Dimethyl 2-sulfanylidenebutanedioate:

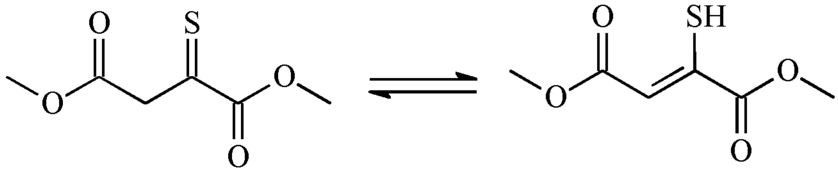

59. Diethyl 2-(hydroxyimino)butanedioate:

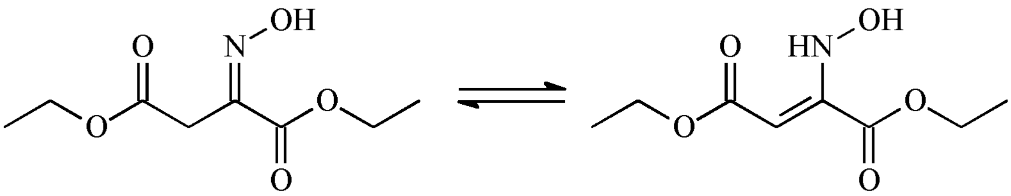

60. 1-Ethyl 4-methyl 2-hydrazinylidenebutanedioate:

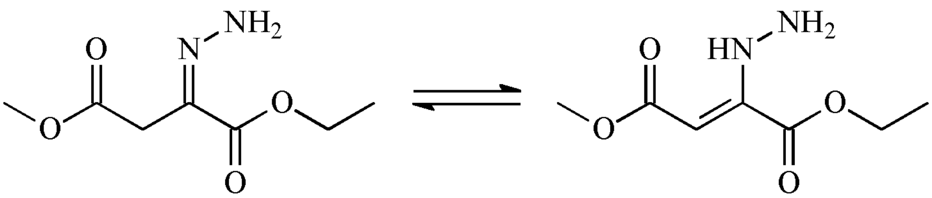

61. 2-aminobut-2-enediamide:

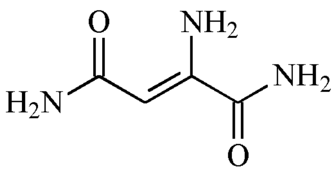

62. Ethyl 2-amino-4-oxo-4-phenylbut-2-enoate:

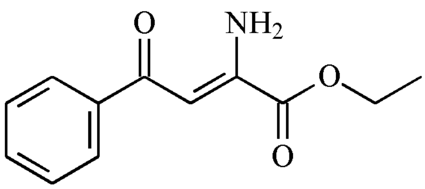

63. Methyl 2-amino-4-(4-chlorophenyl)-4-oxobut-2-enoate:

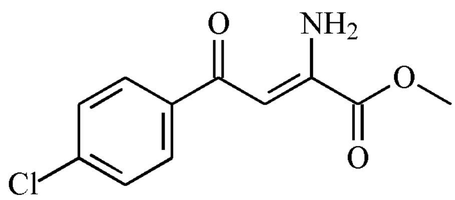

64. Ethyl 2-amino-4-(2-methoxyphenyl)-4-oxobut-2-enoate:

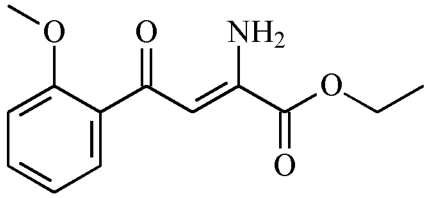

65. Sodium 2-amino-4-(1*H*-indol-3-yl)-4-oxobut-2-enoate:

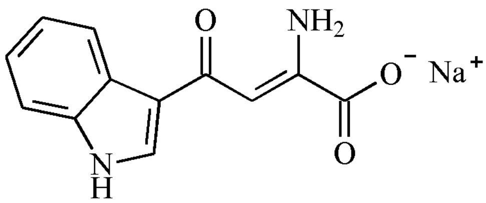

66. Ethyl 2-amino-6-(4-chlorophenyl)-4-oxohexa-2,5-dienoate:

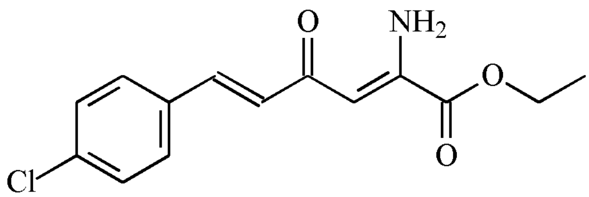

67. Dipropyl 2-aminobut-2-enedioate:

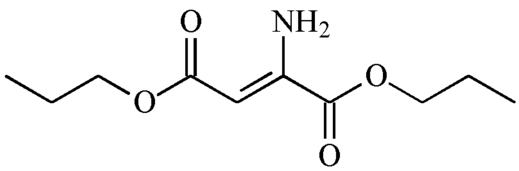

68. Dibutyl 2-aminobut-2-enedioate:

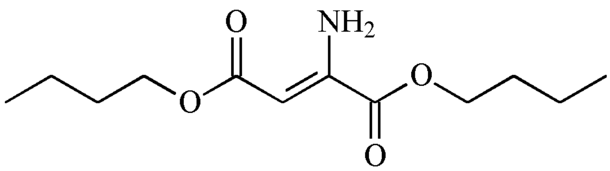

69. Dibenzyl 2-aminobut-2-enedioate:

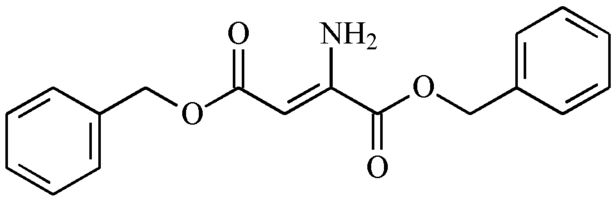

70. Methyl 2-amino-4-oxopent-2-enoate:

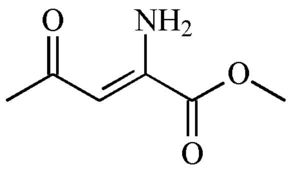

Derivatives of the present disclosure as broadly contemplated herein may be variously specified as excluding any one or more of the following compounds:

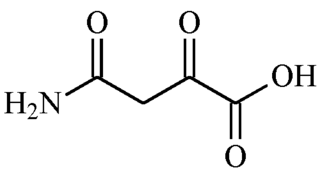
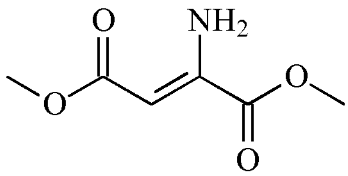
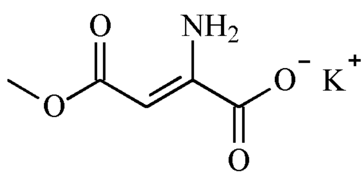

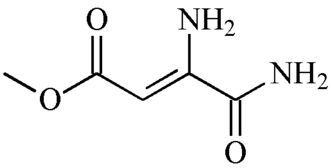
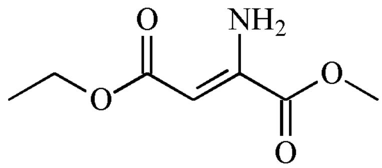

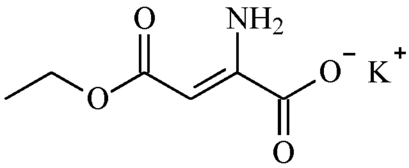
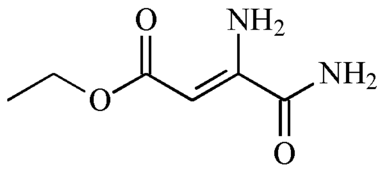

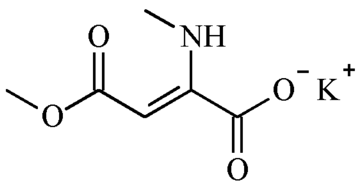
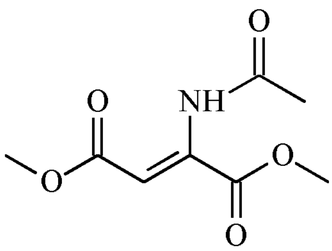
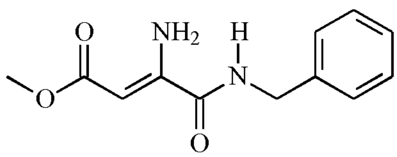

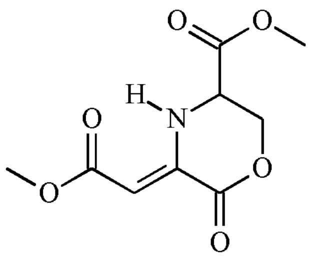

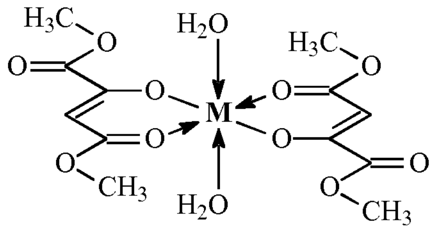

M = Zn, Ni, Mn

It will be appreciated that the plant growth regulator compositions of the disclosure comprise one or more of the compounds of the disclosure, and that the compositions of the disclosure can in specific embodiments comprise different oxaloacetic acid esters, salts and/or derivatives thereof having phytologically beneficial plant growth regulator characteristics. For example, the plant growth regulator composition may comprise two or more different oxaloacetic acid esters. In another embodiment, the plant growth regulator composition may comprise an oxaloacetic acid ester and an oxaloacetic acid ester salt in combination with one another. In still another embodiment, two or more different oxaloacetic acid derivatives may be employed in combination with one another in the composition. It will be appreciated that the compositions of the present disclosure contemplate all permutations of one or more active ingredients selected from the group consisting of oxaloacetic acid esters, salts, and/or derivatives thereof having phytologically beneficial plant growth regulator characteristics.

It will also be recognized that one or more of the compounds of the present disclosure may in various embodiments be utilized in a neat solid or liquid (or oil) form, e.g., as a particulate material that can be applied to a plant or a locus containing plant(s) by dusting or by spray of particulate compounds to such plants, seeds or plant locus, or other solids or liquids broadcasting or administration methods, but more typically the compositions of the present disclosure comprise one or more compounds of the present disclosure, together with a carrier or delivery medium.

The carrier or delivery medium may comprise a solvent or suspension (emulsion) medium in which the compound(s) of the composition are dissolved or suspended (emulgated). For example, the carrier or delivery medium may comprise an aqueous or non-aqueous solvent or solvent mixture, which is solvatingly or suspendingly (emulsifyingly) efficacious as a vehicle for delivery of the compound(s) to a plant, seeds or plant locus. The carrier or delivery medium may therefore be a liquid, gaseous, or biphasic fluid medium. Illustrative carrier or delivery media include solvents such as water, dimethyl sulfoxide (DMSO), and plant and animal oils, and any other fluids, solids, sols, etc. that are compatible with the plant growth regulator compound(s) of interest.

It is a substantial advantage of the plant growth regulator compounds and compositions of the present disclosure that the plant growth regulator compounds thereof can be used to good advantage at very low concentrations in relation to typical plant growth regulator active ingredients of the prior art. For example, in plant growth regulator compositions of the disclosure comprising a carrier material, the concentration of the plant growth regulator compound in the composition may be in a range of from $10^{-11}$ to $10^{-3}$ Molar (M). In various embodiments, such concentration may be in a range of from $10^{-11}$ to $10^{-6}$ M, and in other embodiments the concentration of the plant growth regulator compound(s) in the composition may be in a range of from $10^{-11}$ to $10^{-8}$ M.

The plants to which the plant growth regulator compounds are administered, either by direct application to the plant or a portion thereof, or to seeds, or to a locus containing the plant, such as soil, hydroponic medium, or other environmental material, can be of any suitable plant type, e.g., grains, legumes, fiber producing plants, oil producing plants, tuber producing plants, starch producing plants, grasses, vines, fruits, vegetables, flowering plants, and trees. Illustrative plant species can include, without limitation, sunflower, soybean, alfalfa, cucumber, tomato, wheat, corn, cotton, rice, pumpkin, sugarcane, potato, barley, coffee, bean, kidney bean, soybean, French bean, runner bean, haricot, peas, chickpea, lentil, millet, onion, clover, melilot, batata, yam, Jerusalem artichoke (Helianthus tuberosus), cassava, artichoke, oat, rice, peanut, maize, sorghum, radish, horse, turnip, carrot, canola, rapeseed, flax, sesame, asparagus, lettuce, legume, grape, berry, vine, orange, nut, coconut tree, tobacco, pepper, red pepper, sweet pepper, mustard, buckwheat, eggplant, vegetable marrow, zucchini, melon, watermelon, pineapple, banana, papaya, avocado, kiwi, panicgrass, beet, dill, fennel, mint, cabbage, nappacabbage, cilantro, ginger, parsley, spinach, ruccola, celery, Brussel sprouts, mango, strawberry, cauliflower, oilpalms (Elaeis), hops, cannabis, cocoabean, salvia, aster, China aster (Callistephus chinensis), dragon flowers (Antirrhinum), carnation (Dianthus caryophyllus), rose, dahlia, chrysanthemum, tulpin, narcissus, delphinium, iris, clematis, peony, phlox, rhododendron, hyacinthus, cyclamen, petunia, lilium, and orchids.

The application of the plant growth regulator composition to the plant, seeds or locus of the plant for which the compound(s) of the composition is/are phytologically effective can be carried out in any suitable manner. Illustrative methods of application include, without limitation, spraying, dusting, dipping, drenching, aerosolizing, particulate broadcasting, and placement of a plant growth regulator compound-impregnated article or body in proximity to the plant(s) for out-leaching, exudation, or other emergence of the plant growth regulator compound to the plant, seeds or plant locus. In application to trees, for example, a paste may be formulated containing the plant growth regulator compound, and applied to wounds or otherwise damaged areas of the tree to effect healing, or to effect treatment during ingrafting.

Thus, the plant growth regulator compounds utilized as active ingredients in plant growth regulator compositions of the present disclosure include oxaloacetic acid esters, and salts and derivatives thereof having phytologically beneficial plant growth regulator characteristics, wherein the oxaloacetic acid esters have the formula

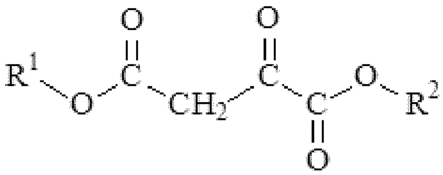

wherein $R^1$ and $R^2$ are each independently selected from $C_1$-$C_{18}$ alkyl,
and wherein the concentration of plant growth regulator compound(s) in the composition is in a range of from $10^{-11}$ to $10^{-3}$ M.

The plant growth regulator compounds (active ingredients) and compositions of the present disclosure provide plant growth regulators that exhibit improved effectiveness of plant growth stimulation, in relation to typical plant growth regulators of the prior art, as a result of being capable of use at very low concentrations and in very small amounts.

In various embodiments, the plant growth regulator compounds (active ingredients) of the present disclosure may be employed in an aqueous solution to constitute a composition containing as the active ingredient the ester of oxaloacetic acid of general form:

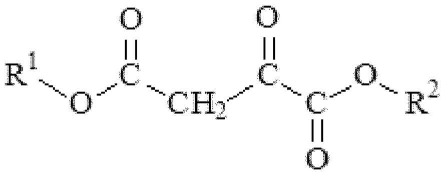

,

where $R^1$ and $R^2$ were independently selected from a series of $C_1$-$C_{18}$-alkyl groups, or its salt, or a mixture thereof, and wherein the active ingredient content corresponds to a concentration from $10^{-11}$ M to $10^{-3}$ M.

The active ingredient of the plant growth regulator in the compositions of the present disclosure is an ester of oxaloacetic acid of general form:

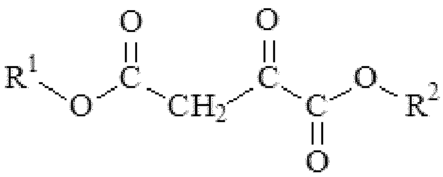

,

wherein $R^1$ and $R^2$ are each independently selected from $C_1$-$C_{18}$-alkyl groups, or its salt, or a derivative thereof, or a mixture thereof.

The plant growth regulator composition with the plant growth regulator compound as the active ingredient therein can be an aqueous solution of the active ingredient compound, or solution of the compound in dimethyl sulfoxide, or in another organic solvent, or a solution or suspension in oil, or in any other liquid, gel, emulsion, paste, resin, powder, wettable powder, granulesor other form. The preparative forms should preferably be selected so as to optimize the effect regulating plant growth. For such purpose, the content of active ingredient compound(s) in plant growth regulator compositions are suitably at a concentration from $10^{-11}$ to $10^{-3}$ M. Methods and materials for manufacture of plant growth regulator compositions in the form of pastes, solutions, emulsions, gels, resins, powders, and others are well known to those of ordinary skill in the art.

The plant growth regulator compositions of the present disclosure can optionally contain organic solvent, which volume is sufficient for initial dissolution of its active ingredient compound(s). Initially, it may be convenient to dissolve the active ingredient(s) in organic solvent, and then to dilute it with water to achieve the desired concentration. This simplifies the preparation of an aqueous solution composition. A preferred organic solvent for the plant growth regulator compounds of the present disclosure comprises dimethyl sulfoxide. It is known that dimethyl sulfoxide increases permeability of biological membranes, enhancing transport of biologically active substances into cells. This effect can further improve the efficiency of the active ingredient compounds of the present disclosure.

The plant growth regulator compounds of the present disclosure may be utilized at fairly small concentrations as an active ingredient in aqueous solutions. For this reason the plant growth regulator compounds of the present disclosure as active ingredients are convenient for storage, sale, and transport, in the form of concentrated solutions of the active ingredient, meant for further dilution to the desired concentration. Solvents for such concentrated solutions can be of any suitable type, and can for example comprise water, organic solvent (e.g. dimethyl sulfoxide), or mixtures of different solvents. Any convenient concentration of the active ingredient compound in such a concentrated solution is acceptable, e.g., 0.01-99% by weight. The plant growth regulator compositions containing the active ingredient compound(s) can also be distributed, transported, and sold in the form of solid powder, pellets, gel, paste or in any other suitable forms with any active ingredient content, e.g., 0.01-99%. The active ingredient compounds can also be distributed in pure form.

The plant growth regulator compositions (as well as the above-mentioned concentrated solutions of the active ingredient compounds) can optionally contain auxiliary agents, e.g., solubilizers, emulsifiers, gelators, spreaders and stickers (i.e., agents, respectively, to facilitate distribution and adhesion), wetting agents, dispersing agents, fixing agents, disintegrating agents, dyes, etc. This amenability to formulation with any of various auxiliary agents makes the utilization of the claimed regulator composition easier and gives it the desired aesthetic properties.

The claimed regulator composition may optionally contain fungicides, plant growth regulators, and/or other pesticides and/or other agrochemicals. This combinatorial approach allows for treatment of plants with all the necessary substances at a time, rather than sequentially, which can significantly simplify and speed up the overall process of plant treatment.

In various embodiments, the active ingredient compounds of the present disclosure may have the form of either an ester of oxaloacetic acid or its salt, or a mixture of an ester of oxaloacetic acid and its salt, in which the active ingredient compound is mostly in three forms, in dynamic equilibrium to each other, namely, ketonic, enolic and enolate anion forms:

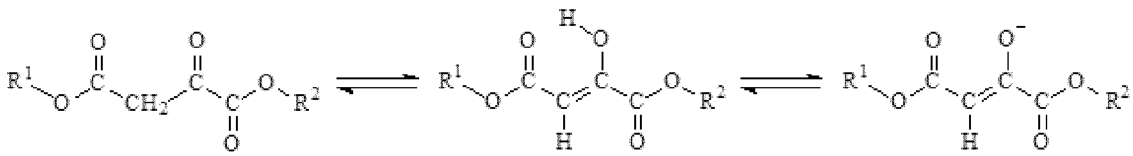

The content of ester of oxaloacetic acid, thus, is the sum of all its forms.

Esters of oxaloacetic acid are CH-acids capable of forming salts (enolates) stable under normal conditions with different cations, such as sodium, potassium, etc. Accordingly, a salt of an ester of oxaloacetic acid can also be used as the active ingredient for the plant growth regulator composition, on an equal basis with an ester of oxaloacetic acid itself. Within the above-discussed range of concentrations from $10^{-11}$ to $10^{-3}$ M, the aqueous solution of a salt of oxaloacetic acid ester is completely equivalent in biological properties at similar concentrations of aqueous solution of the corresponding ester. This is due to the fact, that in diluted aqueous solutions the ester of oxaloacetic acid undergoes acid dissociation and its corresponding salt undergoes hydrolysis. In such case, the ratio of dissociated and non-dissociated forms in the salt solution will be the same as in the ester solution. This relationship can be derived from the equation of the dissociation constant for mono-basic acids (for diluted solutions):

$$\lg ( [A^-] / [AH] ) = pH - pK_a,$$

where $[A^-]$ is an equilibrium concentration of acid anion;
[AH] is an equilibrium concentration of the non-dissociated form of the acid;
pH is a hydrogen index;
$pK_a$ is a negative common logarithm of the acid dissociation constant.

It is clear, that the $[A^-]$ to [AH] ratio, in fact, is only a function of the pH of the solution ($pK_a$ is constant). In the proposed low concentrations of the biologically active ingredient, this ingredient itself leaves pH unaffected. Therefore, the form of substance to be taken for aqueous solution, whether CH-acid or salt, is of no importance and the final content

in the various forms will be equal (ceteris paribus).

A similar effect would obtain for a mixture of the oxaloacetic acid ester and its salt in any ratio.

The same conclusions will apply to plant growth regulator compositions on the basis of the active ingredient compounds in the form of non-aqueous solutions and on regulator compositions with solid media. Accordingly, after treatment with these regulator compositions, the active ingredient compound will penetrate into internal water (aqueous) environment of a plant, where it will exist in the form of an aqueous solution.

The possibility of direct use of salts of esters of oxaloacetic acid greatly simplifies the synthesis of the active ingredient and production of the relevant plant growth regulator composition. As a salt of oxaloacetic acid ester, the plant growth regulator composition may contain a sodium salt of dimethyl oxalacetate, a sodium salt of diethyl oxalacetate, a potassium salt of diethyl oxalacetate, etc., including any other salt with such cation, which does not form water stable chelate complexes with anions of oxaloacetic acid ester.

It is preferable in many applications to use a sodium salt of oxaloacetic acid ester as the active ingredient in the plant growth regulator composition. This simplifies the synthesis of the active ingredient, since these salts deposit in the reaction mixture in the form of sediment that is easy to filter, rinse and dry. In such manner the final product of acceptable purity is obtained in the form of solid powder, which also simplifies the storage, transportation and packaging of the active substance, while ensuring long shelf life.

Synthesis of oxaloacetic acid esters and their sodium salts can be performed by various methods, including for example the synthesis disclosed in Wislicenus W., Grossmann A., Liebigs Annalen der Chemie, vol. 277, (1893) p. 375-383:

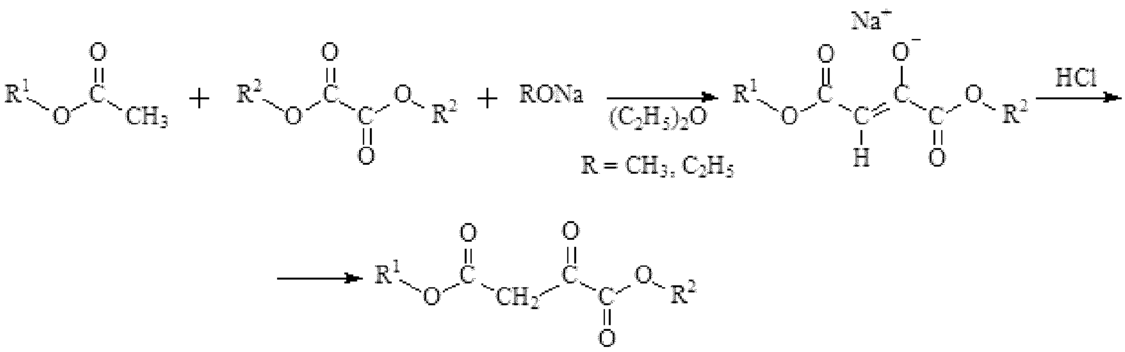


Other salts with any other suitable cations can either be obtained by interaction of oxaloacetic acid ester with an appropriate base, or by interaction of the sodium salt of oxaloacetic acid ester with an ion exchange resin, or by other suitable method.

The oxaloacetic acid derivatives discussed herein can also be synthesized by known methods. Many of these compounds have been synthesized and described, for example compounds 1-70.

The plant growth regulator compositions on the basis of the active ingredient compounds of the present disclosure can be applied in various ways, such as: by spraying seed grains with the growth regulator composition; by immersion of seed grains in the growth regulator composition; by spraying plants or their parts in growth or resting phase; by full or partial immersion of the whole plant or its individual parts in the growth regulator composition; by immersion of root systems of plants, cuttings, inoculants in the growth regulator composition; by injection of growth regulator composition into the plants; by watering of soil with the growth regulator composition; by adding growth regulator compounds or its solutions into resins, waxes, pastes, gels, plasticines, putties, tree-pruning pastes, paints, fertilizers, agrochemicals, pesticides and other substances in contact with plants or otherwise.

Use of $C_1$-$C_{18}$-alkyl groups in the oxaloacetic acid esters is highly advantageous. The plant growth regulator composition and the active ingredient compound(s) perform their function in the entire selected range of alkyl groups, since all the substances of this range are homologous compounds containing inherently similar alkyl substituting groups.

It has been discussed hereinabove that the content of the active ingredient compound in the plant growth regulator composition is from $10^{-11}$ to $10^{-3}$ M. This is due to the fact that throughout the range from $10^{-11}$ M to $10^{-3}$ M, as empirically verified, growth stimulating effects are achieved.

The compounds and compositions of the present disclosure have significant features in relation to plant growth regulator compounds and compositions of the prior art.

The application of oxaloacetic acid is effective as a buffer to reduce the impact of pests and pathogens, to improve metabolism of plants through effects on transamination of alpha-ketoacids and hydroxydicarboxylic acids, as a sterilizer and for bacteriostatic effect, as an intensifier of plant growth regulators, and in other ways. Thus, oxaloacetic acid may be used as an additive to the main component (the growth stimulator active ingredient), improving its properties,

but it is not known that oxaloacetic acid itself shows plant growth stimulant properties, namely, contributes to improving the germination of seeds and germinating power, elongation of the stem and the taproot, strengthening of lateral roots and yield increase.

Existing prior art shows that typical plant growth simulators are used at fairly high concentrations, much higher than the most efficient concentrations of the plant growth regulator compounds of the present disclosure. At the low concentrations utilized for application of the plant growth regulator compositions of the present disclosure, oxaloacetic acid derivatives are not expected to show growth stimulating properties. This results from the fact that oxaloacetic acid and its amide (ketosuccinamate) are important participants in the metabolism of plants, and they are naturally contained in the tissues of plants in high concentrations. Thus, it is non-obvious to the ordinarily skilled persons in the art that the active ingredient compounds of the present disclosure and the plant growth regulator composition on its basis show growth stimulating properties in the very low concentrations taught herein. In addition, the known prior art active ingredient compositions exhibit quite a narrow range of growth stimulating effects.

There is no evidence known to the present inventor that oxaloacetic acid ester or its salts were ever utilized as active ingredients of plant growth regulator compositions.

Application of the active ingredient compounds of the present disclosure, e.g., in an aqueous solution of oxaloacetic acid ester or its salts, or mixtures of this ester and salt in the aforementioned low concentrations achieves an unobvious technical result in markedly enhancing plant growth stimulation efficiency at low concentrations of the active ingredient compounds. Compared to conventional plant growth regulator compounds and compositions, the compounds and compositions of the present disclosure have a remarkably wider range of growth stimulating properties.

The plant growth regulator compositions of the present disclosure can be easily applied using well-known application tools and procedures, to achieve a highly beneficial phytological effect.

The features and advantages of the compounds and compositions of the present disclosure are more fully shown and appreciated, with respect to the following nonlimiting examples.

EXAMPLES

**Example 1.**

Sodium salt of dimethyl ester of oxaloacetic acid (DMOA) and the sodium salt of diethyl ester of oxaloacetic acid (DEOA) were obtained in the Federal State Budgetary Scientific Institution "Institute of Organic Chemistry" named after N.D. Zelinsky of Russian Academy of Sciences (Moscow) in the following way. The 250 ml flask was filled while stirring with 50 mmol of sodium methoxide or sodium ethoxide and a mixture of 100 ml of diethyl ether with 50 mmol of dimethyl oxalate or diethyl oxalate, respectively. Then 51 mmol of methyl acetate or ethyl acetate were added. Then the reaction mixture was boiled within 1 hour with back flow condenser. After cooling, white precipitate sedimented in the form of sodium salt of dimethyl ester of oxaloacetic acid or sodium salt of diethyl ester of oxaloacetic acid, respectively. The resulting salt was filtered, washed with diethyl ether and dried.

The resultant sodium salt of dimethyl ester of oxaloacetic acid and sodium salt of diethyl ester of oxaloacetic acid have the following properties:

1) Properties of the resultant sodium salt of dimethyl ester of oxaloacetic acid. Product yield - 6.83g (75%). $^1$H-NMR (300 MHz, DMSO-$d_6$), $\delta$ (ppm): 3.44 (s, 3H, $CH_3$), 3.61 (s, 3H, $CH_3$), 5.10 (s, 1H, CH). $^{13}$C-NMR (300 MHz, DMSO-$d_6$), $\delta$ (ppm): 48.96 ($CH_3$), 51.38 ($CH_3$), 82.35 (CH), 167.77 (CO), 168.36 (CO), 169.96 (CO).
2) Properties of the resultant sodium salt of diethyl ester of oxaloacetic acid. Product yield - 7,36g (70%). mp = 188-190 °C. $^1$H-NMR(300 MHz, DMSO-$d_6$), $\delta$ (ppm): 1.13 (t, 3H, $CH_3$, $J^3$ = 7 Hz), 1.20 (t, 3H, $CH_3$, $J^3$ = 7 Hz), 3.92 (q, 2H, $CH_2$, $J^3$ = 7 Hz), 4.07 (q, 2H, $CH_2$, $J^3$ = 7 Hz), 5.09 (s, 1H, CH). $^{13}$C-NMR (300 MHz, DMSO-$d_6$), $\delta$ (ppm): 13.92 ($CH_3$), 14.62 ($CH_3$), 56.71 ($CH_2$), 59.97 ($CH_2$), 82.76 (CH), 167.41 (CO), 168.59 (CO), 169.66 (CO).

The above synthesis procedure is easy to implement, comprehensive and easily scalable to industrial production. It uses cheap available reagents and solvents, simple equipment. The end product sediments as a solid precipitate, which is easily filtered, washed and dried to obtain a substance of acceptable purity and high yield. This makes separation and cleaning easier. This determines the high availability and low cost of the claimed regulator and the active ingredient.

**Example 2.**

DMOA was synthesized in the Federal State Budgetary Scientific Institution "Institute of Organic Chemistry" named after N.D. Zelinsky of Russian Academy of Sciences (Moscow) according to the procedure in the Example 1. DEOA used for the tests was purchased from Acros Organics BVBA.

After that DMOA and DEOA were separately dissolved in dimethyl sulfoxide to give solutions of 10 g/l concentration, then water was added to give aqueous solutions of DMOA with concentrations of $10^{-3}$ M, $5.5 \cdot 10^{-5}$ M, $5.5 \cdot 10^{-6}$ M, $5.5 \cdot 10^{-7}$ M, $5.5 \cdot 10^{-8}$ M, $5.5 \cdot 10^{-9}$ M, $10^{-11}$ M and DEOA with concentrations of $10^{-3}$ M, $4.8 \cdot 10^{-5}$ M, $4.8 \cdot 10^{-6}$ M, $4.8 \cdot 10^{-7}$ M, $4.8 \cdot 10^{-8}$ M, $4.8 \cdot 10^{-9}$ M, $10^{-11}$ M.

The obtained aqueous solutions of DMOA and DEOA were tested in the Scientific Center of Soil-Ecological Reasearch of the Federal State-Funded Educational Institution of Higher Education "Russian State Agrarian University - Moscow Agricultural Academy named after K.A. Timiryazev" (Moscow, 2015) according to the method described by T.A. Sergeeva (Method of laboratory testing of herbicides, Plant protection, 1963, No. 2, pp. 42-43). The primary laboratory screening of DMOA and DEOA was carried out on the seeds of five different crops: sunflower of "Varonezh 638" variety, soybean of "Vilana" variety, alfalfa of "Vega" variety, cucumber of "Nezhinsky" variety and tomato of "Volgogradets" variety.

Water was used as a reference.

A single experiment was carried out in a Petri dish. A circle of filter paper was laid at the bottom of the dish and treated with 5 ml of the work solution selected for the experiment. After that 25 seeds were uniformly laid on the surface of the treated filter paper. The Petri dish was kept at a temperature of 21-23 °C with illumination by fluorescent lamps for 7 days. The fourfold replication of the experiment within a single variant was applied.

The experiment identified the number and percentage of normally germinated seeds on day 3 (germinative energy) and on day 7 (germinative capacity) after the beginning of the experiment. On day 7 the main biometric parameters were also measured: sprout height (mm), taproot length (mm) and number of lateral roots (pcs). The experiment results are given in tables 1-5:

Table 1 - Results of laboratory tests of DMOA and DEOA for tomato seeds.
Table 2 - Results of laboratory tests of DMOA and DEOA for sunflower seeds.
Table 3 - Results of laboratory tests of DMOA and DEOA for alfalfa seeds.
Table 4 - Results of laboratory tests of DMOA and DEOA for soybean seeds.
Table 5 - Results of laboratory tests of DMOA and DEOA for cucumber seeds.

Table 1

| Growth Regulator | Concentration, M | Germinative energy, % | Germinative capacity, % | Sprout height, mm | Taproot length, mm | Number of lateral roots, pcs. |
|---|---|---|---|---|---|---|
| Reference (water) | | 79 | 81 | 25.8 | 75.3 | 0.2 |
| DMOA | $10^{-3}$ | 90 | 93 | 25.7 | 89.2 | 0.5 |
| | $5,5 \cdot 10^{-5}$ | 93 | 95 | 25.9 | 83.2 | 0.2 |
| | $5,5 \cdot 10^{-6}$ | 88 | 90 | 24.6 | 79.8 | 0.5 |
| | $5,5 \cdot 10^{-7}$ | 88 | 98 | 23.7 | 74.5 | 0.6 |
| | $5,5 \cdot 10^{-8}$ | 98 | 98 | 23.4 | 83.6 | 0.1 |
| | $5,5 \cdot 10^{-9}$ | 88 | 90 | 24.4 | 86.9 | 0.7 |
| | $10^{-11}$ | 89 | 91 | 23.9 | 85.0 | 0.9 |
| DEOA | $10^{-3}$ | 96 | 97 | 25.4 | 81.1 | 0.7 |
| | $4,8 \cdot 10^{-5}$ | 93 | 95 | 25.3 | 80.1 | 0.5 |
| | $4,8 \cdot 10^{-6}$ | 75 | 80 | 25.7 | 77.7 | 0.2 |
| | $4,8 \cdot 10^{-7}$ | 88 | 91 | 26.3 | 84.6 | 0.4 |
| | $4,8 \cdot 10^{-8}$ | 74 | 76 | 22.7 | 77.9 | 0.2 |
| | $4,8 \cdot 10^{-9}$ | 82 | 89 | 21.3 | 77.9 | 0.4 |
| | $10^{-11}$ | 80 | 85 | 22.2 | 81.0 | 0.5 |

Table 2

| Growth Regulator | Concentration, M | Germinative energy, % | Germinative capacity, % | Sprout height, mm | Taproot length, mm | Number of lateral roots, pcs. |
|---|---|---|---|---|---|---|
| Reference (water) | | 78 | 84 | 20.9 | 39.6 | 8.5 |

(continued)

| Growth Regulator | Concentration, M | Germinative energy, % | Germinative capacity, % | Sprout height, mm | Taproot length, mm | Number of lateral roots, pcs. |
|---|---|---|---|---|---|---|
| DMOA | $10^{-3}$ | 91 | 94 | 24.5 | 50.3 | 10.5 |
| | $5{,}5\cdot10^{-5}$ | 80 | 90 | 23.8 | 35.2 | 9.7 |
| | $5{,}5\cdot10^{-6}$ | 83 | 93 | 23.0 | 46.4 | 8.5 |
| | $5{,}5\cdot10^{-7}$ | 90 | 93 | 26.9 | 29.8 | 8.2 |
| | $5{,}5\cdot10^{-8}$ | 93 | 91 | 28.8 | 38.1 | 7.8 |
| | $5{,}5\cdot10^{-9}$ | 87 | 93 | 27.8 | 51.9 | 11.6 |
| | $10^{-11}$ | 93 | 95 | 27.1 | 47.8 | 11.0 |
| DEOA | $10^{-3}$ | 91 | 99 | 51.2 | 41.7 | 13.9 |
| | $4{,}8\cdot10^{-5}$ | 87 | 93 | 40.1 | 38.4 | 9.6 |
| | $4{,}8\cdot10^{-6}$ | 80 | 90 | 29.7 | 44.0 | 8.9 |
| | $4{,}8\cdot10^{-7}$ | 93 | 93 | 29.2 | 37.0 | 6.9 |
| | $4{,}8\cdot10^{-8}$ | 97 | 99 | 27.7 | 56.3 | 11.2 |
| | $4{,}8\cdot10^{-9}$ | 81 | 87 | 30.5 | 38.3 | 9.5 |
| | $10^{-11}$ | 80 | 84 | 28.6 | 46.2 | 10.5 |

Table 3

| Growth Regulator | Concentration, M | Germinative energy, % | Germinative capacity, % | Sprout height, mm | Taproot length, mm | Number of lateral roots, pcs. |
|---|---|---|---|---|---|---|
| Reference (water) | | 81 | 91 | 27.6 | 31.2 | 0.1 |
| DMOA | $10^{-3}$ | 96 | 100 | 30.9 | 33.4 | 0.6 |
| | $5{,}5\cdot10^{-5}$ | 93 | 100 | 30.0 | 28.1 | 0.6 |
| | $5{,}5\cdot10^{-6}$ | 88 | 100 | 29.0 | 30.9 | 0.7 |
| | $5{,}5\cdot10^{-7}$ | 88 | 93 | 28.9 | 31.3 | 0.6 |
| | $5{,}5\cdot10^{-8}$ | 98 | 93 | 28.9 | 31.2 | 0.5 |
| | $5{,}5\cdot10^{-9}$ | 88 | 100 | 30.1 | 35.6 | 0.6 |
| | $10^{-11}$ | 94 | 99 | 31.2 | 37.2 | 0.7 |
| DEOA | $10^{-3}$ | 95 | 100 | 35.4 | 45.2 | 0.2 |
| | $4{,}8\cdot10^{-5}$ | 93 | 97 | 34.9 | 41.9 | 0.1 |
| | $4{,}8\cdot10^{-6}$ | 73 | 80 | 32.5 | 37.5 | 0.0 |
| | $4{,}8\cdot10^{-7}$ | 88 | 93 | 33.5 | 32.2 | 0.5 |
| | $4{,}8\cdot10^{-8}$ | 73 | 97 | 32.4 | 42.3 | 0.1 |
| | $4{,}8\cdot10^{-9}$ | 80 | 87 | 28.5 | 34.6 | 0.7 |
| | $10^{-11}$ | 83 | 88 | 28.8 | 42.1 | 0.6 |

Table 4

| Growth Regulator | Concentration, M | Germinative energy, % | Germinative capacity, % | Sprout height, mm | Taproot length, mm | Number of lateral roots, pcs. |
|---|---|---|---|---|---|---|
| Reference (water) | | 69 | 87 | 15.4 | 38.5 | 0.2 |

(continued)

| Growth Regulator | Concentration, M | Germinative energy, % | Germinative capacity, % | Sprout height, mm | Taproot length, mm | Number of lateral roots, pcs. |
|---|---|---|---|---|---|---|
| DMOA | $10^{-3}$ | 85 | 98 | 18.9 | 44.8 | 0.5 |
| | $5,5 \cdot 10^{-5}$ | 78 | 89 | 16.0 | 35.5 | 0.2 |
| | $5,5 \cdot 10^{-6}$ | 82 | 97 | 19.0 | 37.6 | 0.0 |
| | $5,5 \cdot 10^{-7}$ | 65 | 90 | 19.0 | 39.5 | 0.0 |
| | $5,5 \cdot 10^{-8}$ | 77 | 87 | 19.5 | 38.4 | 0.0 |
| | $5,5 \cdot 10^{-9}$ | 72 | 83 | 17.8 | 37.0 | 0.0 |
| | $10^{-11}$ | 77 | 88 | 17.4 | 43.2 | 0.3 |
| DEOA | $10^{-3}$ | 89 | 98 | 16.9 | 45.1 | 0.7 |
| | $4,8 \cdot 10^{-5}$ | 87 | 93 | 15.3 | 45.3 | 0.3 |
| | $4,8 \cdot 10^{-6}$ | 75 | 87 | 19.0 | 37.7 | 0.6 |
| | $4,8 \cdot 10^{-7}$ | 72 | 90 | 14.7 | 36.2 | 0.3 |
| | $4,8 \cdot 10^{-8}$ | 73 | 93 | 14.3 | 33.6 | 0.4 |
| | $4,8 \cdot 10^{-9}$ | 80 | 99 | 17.1 | 46.5 | 1.2 |
| | $10^{-11}$ | 75 | 100 | 17.4 | 45.0 | 1.0 |

Table 5

| Growth Regulator | Concentration, M | Germinative energy, % | Germinative capacity, % | Sprout height, mm | Taproot length, mm | Number of lateral roots, pcs. |
|---|---|---|---|---|---|---|
| Reference (water) | | 84 | 91 | 19.5 | 78.2 | 13.6 |
| DMOA | $10^{-3}$ | 87 | 100 | 23.5 | 86.1 | 15.6 |
| | $5,5 \cdot 10^{-5}$ | 86 | 82 | 19.0 | 74.2 | 11.9 |
| | $5,5 \cdot 10^{-6}$ | 88 | 91 | 15.9 | 75.6 | 12.3 |
| | $5,5 \cdot 10^{-7}$ | 85 | 94 | 17.7 | 78.1 | 13.7 |
| | $5,5 \cdot 10^{-8}$ | 86 | 78 | 17.1 | 74.8 | 13.5 |
| | $5,5 \cdot 10^{-9}$ | 82 | 95 | 18.2 | 82.5 | 12.9 |
| | $10^{-11}$ | 86 | 95 | 19.6 | 83.2 | 14.9 |
| DEOA | $10^{-3}$ | 89 | 97 | 30.0 | 86.2 | 15.6 |
| | $4,8 \cdot 10^{-5}$ | 84 | 88 | 27.8 | 83.7 | 14.3 |
| | $4,8 \cdot 10^{-6}$ | 80 | 85 | 22.2 | 88.3 | 14.4 |
| | $4,8 \cdot 10^{-7}$ | 84 | 88 | 22.3 | 89.3 | 15.5 |
| | $4,8 \cdot 10^{-8}$ | 88 | 95 | 19.3 | 79.8 | 12.4 |
| | $4,8 \cdot 10^{-9}$ | 91 | 93 | 21.6 | 93.8 | 13.7 |
| | $10^{-11}$ | 90 | 95 | 23.7 | 92.6 | 15.9 |

The results of the experiments show that the aqueous solutions of DMOA and DEOA in the proposed concentrations have an express ability to stimulate germination, root and stem elongation, growth of lateral roots.

**Example 3.**

DMOA was synthesized in the Federal State Budgetary Scientific Institution "Institute of Organic Chemistry" named after N.D. Zelinsky of Russian Academy of Sciences (Moscow) according to the procedure in the Example 1. DEOA used for the tests was purchased from Acros Organics BVBA.

After that DMOA and DEOA were separately dissolved in dimethyl sulfoxide to give solutions of 10 g/l concentration, then water was added to give aqueous solutions of DMOA with concentrations of $10^{-3}$ M, $5.5 \cdot 10^{-8}$ M, $5.5 \cdot 10^{-9}$ M, $1.8 \cdot 10^{-9}$ M, $5.5 \cdot 10^{-10}$ M, $1.8 \cdot 10^{-10}$ M, $1.8 \cdot 10^{-11}$ M, $10^{-11}$ M and DEOA with concentrations of $10^{-3}$ M, $4.8 \cdot 10^{-8}$ M,

$4.8 \cdot 10^{-9}$ M, $1.6 \cdot 10^{-9}$ M, $4.8 \cdot 10^{-10}$ M, $1.6 \cdot 10^{-10}$ M, $1.6 \cdot 10^{-11}$ M, $10^{-11}$ M.

The field tests of the obtained aqueous solutions of DMOA and DEOA on spring wheat of "Lubava" variety were carried out. The tests were carried out in agroclimatic zone I, on the experimental field of the Grain Crops Protection Systems Laboratory of the Agricultural Research Center of Federal State Budgetary Scientific Institution "Moscow Research Institute of Agriculture" "Nemchinovka" "(Moscow Region, Odintsovo district, settlement Novoivanovskaya, 2015) according to the method adopted for registration tests of plant growth regulators (Guidelines for conducting registration tests of new forms of fertilizers, bioregulators and plant regulators. Moscow-Vladimir, 2009, p. 104; The method of state variety testing of agricultural crops. Grain, cereal, grain legumes and forage crops. Issue 1, Moscow, 1985, page 269, Issue 2, Moscow, 1989, p. 194; OST 10-108-87; GOST 26212-91; GOST-91).

The soil of the experimental field is sod-podzolic on clay loam mantle. The thickness of the arable layer was 27-29 cm with humus content up to 2.2%, $pH_{salt}$ about 5.7-5.8, labile phosphorus 145-155 mg / kg and exchange potassium 100-115 mg/kg of soil.

Tests were conducted from April 29 to July 30, 2015. In the first half of the growing period the weather conditions were unfavorable (abundant rainfalls in May and June), which is a significant stress factor.

Control test - without treatment with growth regulators.

Area of experimental plots - 100 $m^2$, accounting area - 50 $m^2$. Fourfold replication of the experiments.

Preceding crop was winter wheat. Immediately after the autumn harvesting of winter crops the area was treated with disc harrow to a depth of 10-12 cm. Then, fall plowing to a depth of 18-20 cm by a reverse plow, harrowing, and moisture conservation were carried out.

Before sowing the test crop, the soil was fertilized with Azofoska in a dose of $N_{64}P_{64}K_{64}$. Date of sowing: April 29, 2015 Seeding rate: 5.0 million of germinated grains per 1 hectare.

Before sowing, all the seeds were treated with "Vincite Forte" fungicide with a consumption rate of 1.0 1 per ton of seeds, as well as the aqueous solution of DMOA or DEOA selected for the experiment with a consumption rate of 10 l per ton of seeds (this aqueous solution was directly used as a liquid for treatment) or without a growth regulator, depending on the experiment. The following concentrations of the test solutions were used for treatment: $10^{-3}$ M, $5.5 \cdot 10^{-8}$ M, $5.5 \cdot 10^{-9}$ M, $5.5 \cdot 10^{-10}$ M and $10^{-11}$ M of DMOA; $10^{-3}$ M, $4.8 \cdot 10^{-8}$ M, $4.8 \cdot 10^{-9}$ M, $4.8 \cdot 10^{-10}$ M and $10^{-11}$ M of DEOA.

At the tillering phase (May 23) the crops were sprayed against weeds with "Linthur" herbicide at a dose of 175.0 g / ha, pesticides "BI-58 Novy" were used against pests at a dose of 0.5 1 / ha, and against the leaf-stems diseases the crops were sprayed at the early ear emergence stage (June 20) with "Alto Super" fungicide at a dose of 0.5 1 / ha together with aqueous solutions of DMOA or DEOA (or without them). Spraying with aqueous solutions of DMOA or DEOA allows to additionally stimulate plant growth. In this case the dosage of aqueous solutions of DMEA or DEOA was 300 1 / ha (these solutions were sprayed directly). Spraying with aqueous solutions of DMEA and DEOA was carried out for all variants, where DMOA and DEOA were used for seed treatment, except for variants with concentrations of $10^{-3}$ M and $10^{-11}$ M. The concentration of the spraying solution was one-thirtieth of the concentration of the solution previously used for treatment of seeds. Accordingly, for DMOA: $1.8 \cdot 10^{-9}$ M, $1.8 \cdot 10^{-10}$ M, $1.8 \cdot 10^{-11}$ M; for DEOA: $1.6 \cdot 10^{-9}$ M, $1.6 \cdot 10^{-10}$ M, $1.6 \cdot 10^{-11}$ M.

Harvesting of experimental plots was carried out on July 30, before harvesting test sheaves of 0.25 $m^2$ of each variant of the experiment were selected for structural analysis.

The results of the analysis are shown in Table 6 - The results of the field tests of DMOA and DEOA on wheat. The table shows concentrations of the solutions used for treatment of seeds in the respective variants.

Table 6.

| Parameters | Reference sample without treating | DMOA | | | | | DEOA | | | | |
|---|---|---|---|---|---|---|---|---|---|---|---|
| | | $10^{-3}$ M | $5.5\text{-}10^{-8}$ M | $5.5\text{-}10^{-9}$ M | $5.5\text{-}10^{-10}$ M | $10^{-11}$ M | $10^{-3}$ M | $4.8\text{-}10^{-8}$ M | $4.8\text{-}10^{-9}$ M | $4.8\text{-}10^{-10}$ M | $10^{-11}$ M |
| Plant height. cm | 98.3 | 104.2 | 101.5 | 105.1 | 104.1 | 103.7 | 105.3 | 101.7 | 99.4 | 104.3 | 101.0 |
| Number of stems. pcs./$m^2$ | 383 | 412 | 420 | 752 | 396 | 387 | 341 | 612 | 408 | 324 | 401 |
| Product ive bushiness | 1.47 | 1.67 | 1.57 | 1.21 | 1.48 | 1.45 | 1.52 | 1.34 | 1.33 | 1.69 | 1.54 |
| Weight of 1 000 seeds. g | 44.56 | 49.37 | 46.22 | 47.07 | 46.51 | 45.97 | 49.26 | 48.15 | 46.88 | 48.38 | 45.45 |
| Main head length, cm | 8.1 | 8.9 | 8.7 | 8.9 | 8.8 | 8.5 | 9.8 | 8.6 | 9.2 | 10.8 | 8.4 |
| Number of grains of main head. pcs. | 33 | 34 | 36 | 37 | 37 | 35 | 40 | 39 | 41 | 51 | 35 |
| Weight of grains of main head. g | 1.57 | 1.78 | 1.76 | 1.79 | 1.71 | 1.65 | 2.07 | 1.91 | 1.96 | 2.65 | 1.67 |
| Protein (N 5,7), % dry basis | 14.12 | 14.95 | 14.88 | 14.92 | 14.73 | 14.58 | 14.17 | 14.88 | 14.95 | 14.14 | 14.23 |
| Gluten, % dry basis | 29.60 | 33.57 | 33.64 | 34.71 | 33.81 | 33.02 | 34.51 | 31.85 | 32.91 | 31.61 | 31.77 |
| Starch, % dry basis | 60.74 | 61,40 | 61.54 | 61.72 | 61.27 | 61.11 | 61.83 | 61.15 | 61.43 | 61.60 | 61.29 |
| Yield. t/ha | 4.88 | 5.84 | 6.44 | 6.49 | 6.41 | 5.32 | 6.12 | 5.50 | 5.32 | 5.00 | 5.29 |
| Yield. % | 100.0 | 119.7 | 131.9 | 133.0 | 131.4 | 109.0 | 125.4 | 112.7 | 109.0 | 102.5 | 108.4 |

The results of the experiments proved that the claimed plant growth regulator and the active ingredient had a positive effect on the wheat yield increase with all tested concentrations. In addition, the claimed plant growth regulator and the active ingredient contributed to a significant increase in the number of productive stems per 1 $m^2$, growth of the grain weight, elongation of the main head, the increase in the quantity and weigh of grains from the main head. At the same time, the content of gluten and starch in wheat increased significantly, and a slight increase in protein content was noticed. There were no negative effects on the wheat plant and on the grain quality indicators.

With results of the experiments it may be concluded that aqueous solutions of DMOA and DEOA in the proposed concentrations have an express ability to stimulate growth, increase yield and quality of wheat, despite the significant stress factor (abundant rainfalls in May and June). At the same time, there was no excessive growth of the plant in height, which could result in drowning.

The Tables 1-6 show that in comparison with prototypes, the active ingredient exerts growth stimulating properties in smaller dosages, which reduces its consumption. Also, the claimed regulator and the active ingredient have qualitative advantages, consisting in expansion of the growth stimulating effects. Namely, the claimed regulator and the active ingredient, unlike the prototypes, allow for taproot elongation, increase in the number of lateral roots, and elongation of the germinant (main stem). In addition, the claimed regulator and the active ingredient are superior to prototype in terms of alfalfa germination index and allow for the increase of not only protein, but also starch content in wheat.

## Example 4

The methodology of this example is applicable to potato and other tuber plants. For the experiment, tubers of the same size and the same physiological state were selected. Then the tubers were dipped for 30 seconds in a container with an aqueous solution of the test plant growth regulator compound at the test concentration. The tubers were dried in the air after treatment and planted in the ground. During the vegetation period, phenological observations are carried out, the germination capacity, the number of main stems, the weight of the tops, roots, and the leaf area are determined. After harvesting, the yields of the tubers, the degree of damage to diseases and pests, as well as the content of dry matter, starch, vitamin C and nitrates are determined.

## Example 5

The methodology of this example is applicable to flowers such as China aster, dragon flowers, and others. Seedlings of flower crops were sprayed with aqueous solutions of the claimed preparations at the tested concentrations. The height of the plants, the number of flowers, the number of lateral peduncles, the diameter of the inflorescences and other parameters influencing the decorative properties of flowers are measured.

The results of the experiments described above confirm that the plant growth regulator compounds and compositions of the present invention achieve a highly beneficial result.

In addition, the use of the active ingredient compounds of the present disclosure, in concentrations in the range of $10^{-11}$ to $10^{-3}$ M achieves an increase in the efficiency of plant growth stimulation with a low dosage of the active ingredient, via strengthening of seed germination power, increased germination, and stimulation of the growth of shoots and roots. Moreover, the plant growth regulator compounds and compositions of the present disclosure contribute to an increase in the yield and quality of the plants treated with such compounds and compositions.

The technical results obtained on a variety of different plants, including one monocotyledonous (wheat) and five dicotyledonous of four different families (tomato, sunflower, soy, alfalfa, cucumber) evidence the utility of the compounds and compositions of the present disclosure to stimulate the growth of plants of all kinds.

The plant growth regulator compounds and compositions of the present disclosure do not contain any dangerous substances. Oxaloacetic acid esters are derived from naturally-occurring oxaloacetic acid (Krebs cycle), and they can easily, quickly and without residue be decomposed by soil microorganisms. With due regard to low dosages of active ingredients, the plant growth regulator compositions of the present disclosure achieve superior phytological effects without any environmentally adverse effects or health or safety issues to persons consuming agricultural plant products treated with such compositions.

Accordingly, although the disclosure has been set forth herein in reference to specific aspects, features and illustrative embodiments, it will be appreciated that the utility of the disclosure is not thus limited, but rather extends to and encompasses numerous other variations, modifications and alternative embodiments, as will suggest themselves to those of ordinary skill in the field of the present disclosure, based on the description herein. Correspondingly, the disclosure as hereinafter claimed is intended to be broadly construed and interpreted, as including all such variations, modifications and alternative embodiments, within its spirit and scope.

The plant growth regulator compounds (active ingredients) and compositions of the present disclosure may be applied to a wide variety of plants or loci containing plants, to mediate phytologically favorable results such as stimulation of plant growth, increases in plant yields, and improvement of the quality of plant products. The plant growth regulator

compounds of the disclosure can be used at concentrations that are remarkably lower than concentrations typically used with plant growth regulator active ingredients of the prior art, so that relatively small applied amounts can be used to great benefit. In addition, the plant growth regulator compounds of the present disclosure are readily biodegradable and are environmentally benign.

## Claims

1. A plant growth regulator composition, **characterized in that** the active ingredient comprises oxaloacetic acid ester or a salt thereof, or a derivative thereof, wherein the oxaloacetic acid ester has the formula:

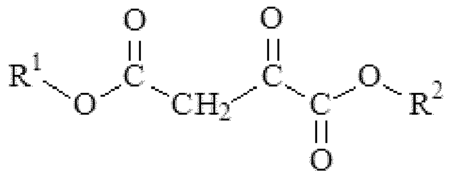

,

wherein $R^1$ and $R^2$ are each independently selected from $C_1$-$C_{18}$-alkyl groups, and wherein the active ingredient content corresponds to a concentration from $10^{-11}$ M to $10^{-3}$ M.

2. The composition of claim 1, wherein the active ingredient comprises a sodium salt of dimethyl oxaloacetate having the following formula:

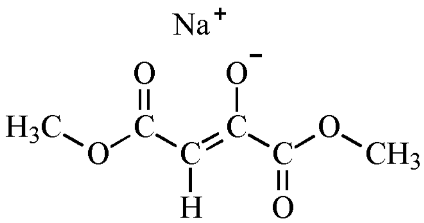

3. The composition of claim 1, wherein the active ingredient comprises a sodium salt of diethyl oxaloacetate having the following formula:

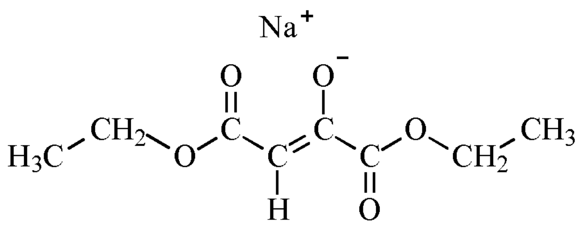

4. The composition of claim 1, further comprising a carrier or delivery medium for the active ingredient.

5. The composition of claim 4, wherein the carrier or delivery medium for the active ingredient comprises an organic solvent.

6. The composition of claim 5, wherein the organic solvent comprises dimethyl sulfoxide.

7. The composition of claim 1, wherein the active ingredient comprises an oxaloacetic acid ester derivative having the following formula:

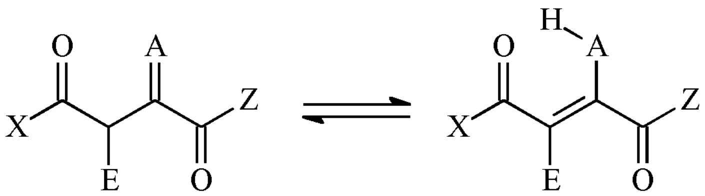

wherein A, E, X and Z are each independently take certain values, namely:
A = O, S, NH, N-OH, N-$NH_2$;
X = OH, $NH_2$, O-R (where R = $C_1$-$C_{18}$-alkyl, $C_1$-$C_{18}$-alkenyl, $C_1$-$C_{18}$-alkynyl, $C_3$-$C_6$-cycloalkyl, wherein one or more hydrogen atoms inside the cycloalkyl may be substituted by $C_1$-$C_4$-alkyl groups, benzyl), NH-R' (wherein R' = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1'}R^{2'}$ (wherein $R^{1'}$ and $R^{2'}$ are independently take values: $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholyl, $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl (including ring substituted by one or more chloro), 2-phenethyl (including ring substituted by one or more chlorine), 2-phenylvinyl (including ring substituted by one or more chloro), naphthyl, indolyl, furanyl, thiophenyl;
Z = OH, $NH_2$, O-R" (where R" = $C_1$-$C_{18}$-alkyl, $C_1$-$C_{18}$-alkenyl, $C_1$-$C_{18}$-alkynyl, $C_3$-$C_6$-cycloalkyl, wherein one or more hydrogen atoms within the cycloalkyl may be substituted by $C_1$-$C_4$-alkyl groups, benzyl), NH-R* (wherein R* = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1''}R^{2''}$ (wherein $R^{1''}$ and $R^{2''}$ each independently take values: $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholinyl;
E = H, CN, halogen (F, Cl, Br, I), O-R'" (wherein R'" = $C_1$-$C_4$-alkyl, allyl, propargyl, $C_3$-$C_6$-cycloalkyl, benzyl), S-R'" (wherein R'" = $C_1$-$C_4$-alkyl, allyl, propargyl, $C_3$-$C_6$-cycloalkyl, benzyl), O-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), S-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl, naphthyl, indolyl, furanyl, tetrahydrofuranyl, thiophenyl, tetrahydrothiophenyl;
also X and E may be connected forming a 5-7-membered cycle through the following bridges (from X-terminus to E-terminus): $-(CH_2)_m-$ (wherein m = 3-5), - $(CH_2)_n$-(C=O)- (where n = 2-4), -O-$(CH_2)_n$- (where n = 2-4), -NH-$(CH_2)_n$- (where n = 2-4), -CH*-$(CH_2)_k$-CH*- (wherein k = 1-2, and CH* carbons are connected to each other via a $-(CH_2)_j-$ bridge, where j = 1-2, regardless k), as any C-C bond within the said bridges may be fuse with benzene ring (wherein the benzene ring may be substituted by one or more identical or different functional groups of the following: methyl, ethyl, propyl, butyl, hydroxy, methoxy, ethoxy, halogen, nitro, trifluoromethyl), also one or more hydrogen atoms within said bridges (including NH, if present) may be further substituted by $C_1$-$C_4$-alkyl group;
and E and Z may be joined to form a 5-6-membered ring through following bridges (from Z-terminus to E-terminus): -NH-$(CH_2)_r$- (wherein r = 1-2), and one or more of the hydrogen atoms inside said bridges (including NH) may be further substituted by $C_1$-$C_4$-alkyl group;
herewith oxaloacetic acid derivatives exclude compounds where A, E, X, Z simultaneously have the following values: A = O, E = H, X = $NH_2$ and Z = OH in the first case; A = NH, E = H, X = Z = $OCH_3$ in the second case; A = NH, E = H, X = $OC_2H_5$ and Z = $OCH_3$ in the third case; A = NH, E = H, X = $OCH_3$, Z = OH/OK in the fourth case; A = NH, E = H, X = $OCH_3$, Z = $NH_2$ in the fifth case; A = NH, E = H, X = $OC_2H_5$, Z = OH/OK in the sixth case; A = NH, E = H, X = $OC_2H_5$, Z = $NH_2$ in the seventh case; A = NH, E = H, X = $OCH_3$, Z = -NH-$CH_2$-Ph (wherein Ph = phenyl) in the eighth case;
herewith derivatives of oxaloacetic acid also include salts and complexes with metals for all permissible compounds mentioned above as suitable derivatives, except chelate complexes consisting of two anions of dimethyl ester of oxaloacetic acid, and zinc / manganese / nickel.

**8.** The composition of claim 1, wherein the active ingredient comprises ethyl 4-(dimethylamino)-2,4 -dioxobutanoate:

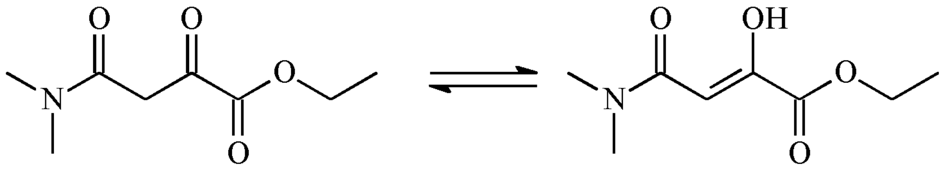

.

**9.** The composition of claim 1, wherein the active ingredient is dissolved in an aqueous medium.

**10.** The composition of claim 1, wherein the active ingredient is provided in a solid carrier or delivery medium.

**11.** A method for treating plants to increase their growth, comprising applying to said plants or to seeds of said plants, or to a locus containing said plants the plant growth regulator composition according to any one of claims 1 to 10.

**12.** The method of claim 11, wherein said plants are selected from the group consisting of grains, legumes, fiber producing plants, oil producing plants, tuber producing plants, starch producing plants, grasses, vines, fruits, vegetables, flowering plants, and trees.

**13.** The method of claim 11, wherein said plants are selected from the group consisting of sunflower, soybean, alfalfa, cucumber, tomato, wheat, corn, cotton, rice, pumpkin, sugarcane, potato, barley, coffee, bean, kidney bean, soybean, french bean, runner bean, haricot, peas, chickpea, lentil, millet, onion, clover, melilot, batata, yam, Jerusalem artichoke (Helianthus tuberosus), cassava, artichoke, oat, rice, peanut, maize, sorghum, radish, horse, turnip, carrot, canola, rapeseed, flax, sesame, asparagus, lettuce, legume, grape, berry, vine, orange, nut, coconut tree, tobacco, pepper, red pepper, sweet pepper, mustard, buckwheat, eggplant, vegetable marrow, zucchini, melon, watermelon, pineapple, banana, papaya, avocado, kiwi, panicgrass, beet, dill, fennel, mint, cabbage, nappacabbage, cilantro, ginger, parsley, spinach, ruccola, celery, Brussel sprouts, mango, strawberry, cauliflower, oilpalms (Elaeis), hops, cannabis, cocoabean, salvia, aster, China aster (Callistephus chinensis), dragon flowers (Antirrhinum), carnation (Dianthus caryophyllus), rose, dahlia, chrysanthemum, tulpin, narcissus, delphinium, iris, clematis, peony, phlox, rhododendron, hyacinthus, cyclamen, petunia, lilium, and orchids.

**14.** The method of claim 11, wherein said plants are wheat plants.

**15.** An active ingredient of a plant growth regulator, said active ingredient being an ester of oxaloacetic acid or salt of thereof, or derivative of thereof, wherein oxaloacetic acid ester has the following formula:

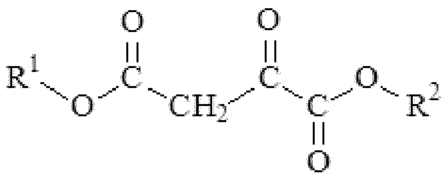

,

where $R^1$ and $R^2$ are each independently selected from $C_1$-$C_{18}$-alkyl groups.

**16.** The active ingredient of claim 15, comprising a sodium salt of dimethyl oxaloacetate.

**17.** The active ingredient of claim 15, comprising a sodium salt of diethyl oxaloacetate.

**18.** The active ingredient of claim 15, comprising an oxaloacetic acid ester derivative having the following formula:

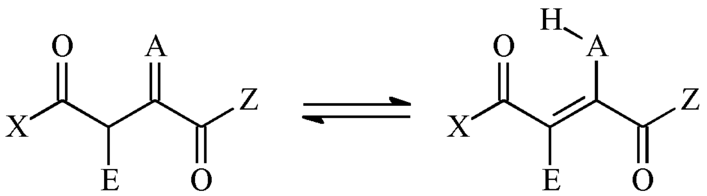

wherein A, E, X and Z are each independently take certain values, namely:
A = O, S, NH, N-OH, N-$NH_2$;
X = OH, $NH_2$, O-R (where R = $C_1$-$C_{18}$-alkyl, $C_1$-$C_{18}$-alkenyl, $C_1$-$C_{18}$-alkynyl, $C_3$-$C_6$-cycloalkyl, wherein one or more hydrogen atoms inside the cycloalkyl may be substituted by $C_1$-$C_4$-alkyl groups, benzyl), NH-R' (wherein R' = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1'}R^{2'}$ (wherein $R^{1'}$ and $R^{2'}$ are independently take values: $C_1$-$C_4$-alkyl, allyl, propargyl,$C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholyl, $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl (including ring substituted by one or more chloro), 2-phenethyl (including ring substituted by one or more chlorine), 2-phenylvinyl (including ring substituted by one or more chloro), naphthyl, indolyl, furanyl, thiophenyl;
Z = OH, $NH_2$, O-R" (where R" = $C_1$-$C_{18}$-alkyl, $C_1$-$C_{18}$-alkenyl, $C_1$-$C_{18}$-alkynyl, $C_3$-$C_6$-cycloalkyl, wherein one or more hydrogen atoms within the cycloalkyl may be substituted by $C_1$-$C_4$-alkyl groups, benzyl), NH-R* (wherein R* = $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), $NR^{1"}R^{2"}$ (wherein $R^{1"}$ and $R^{2"}$ each independently take values: $C_1$-$C_4$-alkyl, allyl, propargyl, $C_3$-$C_6$-cycloalkyl, benzyl, phenyl, tolyl, 2-hydroxyethyl, 2-methoxyethyl, 2-ethoxyethyl), N-pyrrolidinyl, N-piperidinyl, 4-hydroxypiperidinyl, N-morpholinyl;
E = H, CN, halogen (F, Cl, Br, I), O-R'" (wherein R'" = $C_1$-$C_4$-alkyl, allyl, propargyl, $C_3$-$C_6$-cycloalkyl, benzyl), S-R'" (wherein R'" = $C_1$-$C_4$-alkyl, allyl, propargyl, $C_3$-$C_6$-cycloalkyl, benzyl), O-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), S-Ar (where Ar = $C_6$-$C_{14}$-aryl, including ring substituted by one or more identical or different functional groups from the following series: methyl, ethyl, propyl, butyl, hydroxy, methoxy, ethoxy, halogen, nitro, trifluoromethyl), $C_1$-$C_6$-alkyl, $C_1$-$C_6$-alkenyl, $C_1$-$C_6$-alkynyl, $C_3$-$C_6$-cycloalkyl (wherein one or more hydrogen atoms within the cycloalkyl may be substituted by $C_1$-$C_4$-alkyl groups), $C_6$-$C_{14}$-aryl (including ring substituted by one or more identical or different functional groups from the following series: methyl, ethyl, propyl, butyl, hydroxy, methoxy, ethoxy, halogen, nitro, trifluoromethyl), benzyl, naphthyl, indolyl, furanyl, tetrahydrofuranyl, thiophenyl, tetrahydrothiophenyl;
also X and E may be connected forming a 5-7-membered cycle through the following bridges (from X-terminus to E-terminus): $-(CH_2)_m-$ (wherein m = 3-5), - $(CH_2)_n$-(C=O)- (where n = 2-4), -O-$(CH_2)_n$- (where n = 2-4), -NH-$(CH_2)_n$- (where n = 2-4), -CH*-$(CH_2)_k$-CH*- (wherein k = 1-2, and CH* carbons are connected to each other via a $-(CH_2)_j-$ bridge, where j = 1-2, regardless k), as any C-C bond within the said bridges may be fuse with benzene ring (wherein the benzene ring may be substituted by one or more identical or different functional groups of the following: methyl, ethyl, propyl, butyl, hydroxy, methoxy, ethoxy, halogen, nitro, trifluoromethyl), also one or more hydrogen atoms within said bridges (including NH, if present) may be further substituted by $C_1$-$C_4$-alkyl group;
and E and Z may be joined to form a 5-6-membered ring through following bridges (from Z-terminus to E-terminus): -NH-$(CH_2)_r$- (wherein r = 1-2), and one or more of the hydrogen atoms inside said bridges (including NH) may be further substituted by $C_1$-$C_4$-alkyl group;
herewith oxaloacetic acid derivatives exclude compounds where A, E, X, Z simultaneously have the following values: A = O, E = H, X = $NH_2$ and Z = OH in the first case; A = NH, E = H, X = Z = $OCH_3$ in the second case; A = NH, E = H, X = $OC_2H_5$ and Z = $OCH_3$ in the third case; A = NH, E = H, X = $OCH_3$, Z = OH/OK in the fourth case; A = NH, E = H, X = $OCH_3$, Z = $NH_2$ in the fifth case; A = NH, E = H, X = $OC_2H_5$, Z = OH/OK in the sixth case; A = NH, E = H, X = $OC_2H_5$, Z = $NH_2$ in the seventh case; A = NH, E = H, X = $OCH_3$, Z = -NH-$CH_2$-Ph (wherein Ph = phenyl) in the eighth case;
herewith derivatives of oxaloacetic acid also include salts and complexes with metals for all permissible compounds mentioned above as suitable derivatives, except chelate complexes consisting of two anions of dimethyl ester of oxaloacetic acid, and zinc / manganese / nickel.

## INTERNATIONAL SEARCH REPORT

International application No.
PCT/RU 2017/000432

A. CLASSIFICATION OF SUBJECT MATTER
A01N 37/42 (2006.01); A01N 55/02 (2006.01); A01P 21/00 (2006.01)

According to International Patent Classification (IPC) or to both national classification and IPC

B. FIELDS SEARCHED

Minimum documentation searched (classification system followed by classification symbols)
A01N 37/42, 55/02, A01P 21/00

Documentation searched other than minimum documentation to the extent that such documents are included in the fields searched

Electronic data base consulted during the international search (name of data base and, where practicable, search terms used)
PatSearch, esp@cenet, USPTO, Google

C. DOCUMENTS CONSIDERED TO BE RELEVANT

| Category* | Citation of document, with indication, where appropriate, of the relevant passages | Relevant to claim No. |
|---|---|---|
| A | US 2015/0051072 A1 (LOS ALAMOS NATIONAL SECURIT, LLC) 19.02.2015, the claims, the abstract | 1-18 |
| A | UA 19812 A (OBSCHESTVO S OGRANICHENNOI OTVETSTVENNOSTJU NAUCHNO-PROIZVODSTVENNOGO PREDPRIYATIYA "AGRODAR") 25.12.1997 | 1-18 |
| A | CN 103109860 A (ZHONGTAI BRANCH OF ZHEJIANG NEW BLUESKY LANDSCAPE NURSERY STOCK SCIENCE & TECHNOLOGY CO LTD) 22.05.2013 | 1-18 |
| A | US 2004/0171687 A1 (MAURICE C. KEMP et al.) 02.09.2004 | 1-18 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 November 2017 (22.11.2017) | 30 November 2017 (30.11.2017) |
| Name and mailing address of the ISA/ RU | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2184450
- US 20150051072 A


### Non-patent literature cited in the description


- **WISLICENUS W. ; GROSSMANN A.** *Liebigs Annalen der Chemie,* 1893, vol. 277, 375-383